# EUROPEAN PATENT APPLICATION

(11) **EP 4 342 889 A1**
(43) Date of publication of application: **27.03.2024**
(21) Application number: 22845898.0
(22) Date of filing: 19.07.2022
(51) Int. Cl.: C07D 401/04, A61K 31/455, A61P 33/06

(54) **SALT OF HETEROCYCLIC COMPOUND WITH ANTI-MALARIA ACTIVITY, AND CRYSTALS THEREOF**

(30) Priority: 21.07.2021 JP 2021120421
(71) Applicant: Eisai R&D Management Co., Ltd., Tokyo 112-8088 (JP)
(72) Inventor: KUSHIDA Ikuo, Tsukuba-shi, Ibaraki 300-2635 (JP); KOMURA Fusae, Tsukuba-shi, Ibaraki 300-2635 (JP); SHIBUGUCHI Nao, Tsukuba-shi, Ibaraki 300-2635 (JP)
(74) Representative: Hoffmann Eitle
(86) International application number: PCT/JP2022/027971
(87) International publication number: WO 2023/002965

(57) **Abstract**

This salt of the compound represented by formula (1) and crystals thereof are applicable as an active pharmaceutical ingredient of pharmaceutical products.

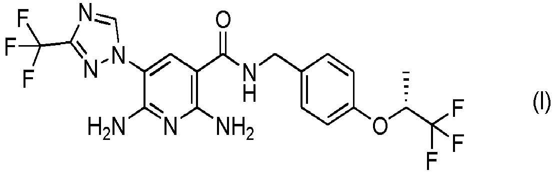

## Description

### Technical Field

The present invention relates to a salt of a heterocyclic compound having antimalarial activity, and a crystal thereof. In addition, the present invention relates to a pharmaceutical composition comprising the salt or the crystal.

### Background Art

Malaria is a life-threating infectious disease caused by Plasmodium falciparum. By estimate, over 200 million people were infected with and over 400 thousand people were killed by malaria in 2018, and most of them are children in African countries. Many countries, companies, and scientists are actively cooperating to eradicate malaria. The current standard of care for treating malaria involves combination therapy with artemisinin. The combination therapy with artemisinin was reported to cause the development of tolerance with high probability (NPL 1 and 2). Thus, a novel compound based on a new mechanism of action has been demanded.

Glycosylphosphatidylinositol (GPI), which is a component possessed by all eukaryotes in common, has a role of anchoring many proteins to the cell surface. Gwt1p, one of essential enzymes in the biosynthesis of GPI, was reported to acylate inositol (see NPL 3 to 6).

In the course of biological studies, it was reported that the GWT1 gene, which encodes the enzyme Gwt1p, is highly conserved among eukaryotes, including Plasmodium falciparum, the pathogen for malaria. A preliminary hit compound with inhibitory activity on plasmodial Gwt1p showed anti-Plasmodium activity in vitro and in vivo. Therefore, a compound that selectively inhibits the biosynthesis of GPI, in particular, the acylation of the inositol ring, possibly serves as an extremely useful antimalarial agent.

PTL 1 is a prior art relating to an antimalarial agent based on such a mechanism. PTL 1 describes heterocyclic compounds having antimalarial activity by inhibition of the biosynthesis of GPI via inhibition of the activity of the GWT1 gene product derived from plasmodia. However, the compounds disclosed in PTL 1 have 2-benzylpyridine as a common structure, and clearly differ structurally from the compound according to the present invention.

PTL 2 is a prior art with the highest structural similarity to the compound according to the present invention. PTL 2 discloses N-unsubstituted diaminopyridine derivatives. However, not only the compound according to the present invention, but 5-substituted diaminopyridine derivatives, are not at all disclosed in PTL 2.

### Citation List

### Patent Literature

[PTL 1] WO 2004/048567
[PTL 2] WO 2006/016548

### Non-Patent Literature

[NPL 1] Yeung S, Socheat D, Moorthy VS et al. Artemisinin resistance on the Thai-Cambodian border. Lancet 2009; 374: 1418-9.
[NPL 2] Hawkes M, Conroy AL, Kain KC. Spread of artemisinin resistance in malaria. The New England journal of medicine 2014; 371: 1944-5.
[NPL 3] Okamoto M, Yoko-o T, Umemura M et al. Glycosylphosphatidylinositol-anchored proteins are required for the transport of detergent-resistant microdomain-associated membrane proteins Tat2p and Fur4p. The Journal of biological chemistry 2006; 281: 4013-23.
[NPL 4] Sagane K, Umemura M, Ogawa-Mitsuhashi K et al. Analysis of membrane topology and identification of essential residues for the yeast endoplasmic reticulum inositol acyltransferase Gwtlp. The Journal of biological chemistry 2011; 286: 14649-58.
[NPL 5] Tsukahara K, Hata K, Nakamoto K et al. Medicinal genetics approach towards identifying the molecular target of a novel inhibitor of fungal cell wall assembly. Mol Microbiol 2003; 48: 1029-42.
[NPL 6] Umemura M, Okamoto M, Nakayama K et al. GWT1 gene is required for inositol acylation of glycosylphosphatidylinositol anchors in yeast. The Journal of biological chemistry 2003; 278: 23639-47.

### Summary of Invention

### Technical Problem

The present inventors have found that a compound represented by the following formula (I) (hereinafter, also referred to as "compound (I)") has antimalarial activity. Thus, compound (I) has a potential use as a prophylactic agent and/or therapeutic agent for malaria.

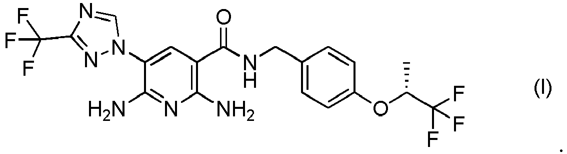

In general, the physical properties of a compound and a crystal thereof to be used as a pharmaceutical have significant influence, for example, on the bioavailability of a drug, the purity of a drug substance, and the formulation of the pharmaceutical preparation. Accordingly, an object of the present specification is to provide pharmaceutically acceptable salts of compound (I) and crystals thereof having a potential use as a drug substance for pharmaceuticals.

### Solution to Problem

In view of those circumstances, the present inventors extensively studied on compound (I), and have found salts of compound (I) and crystals thereof, completing the present specification.

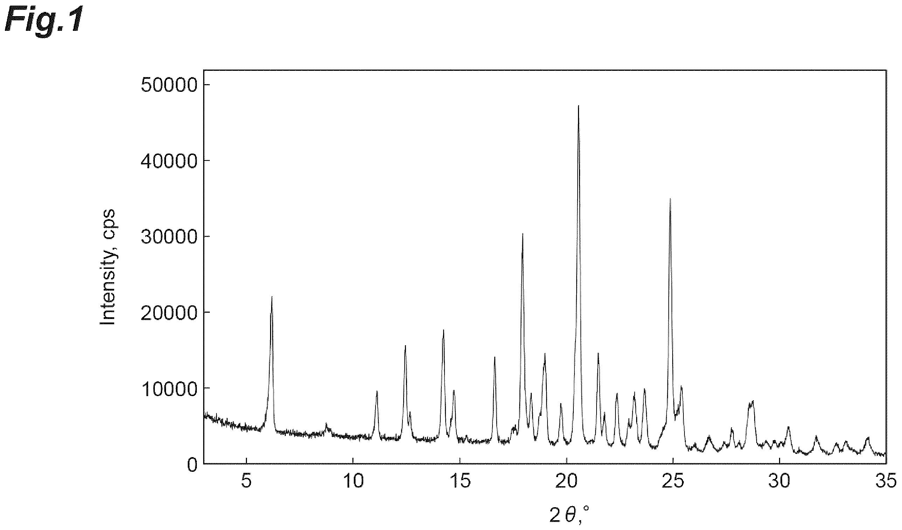

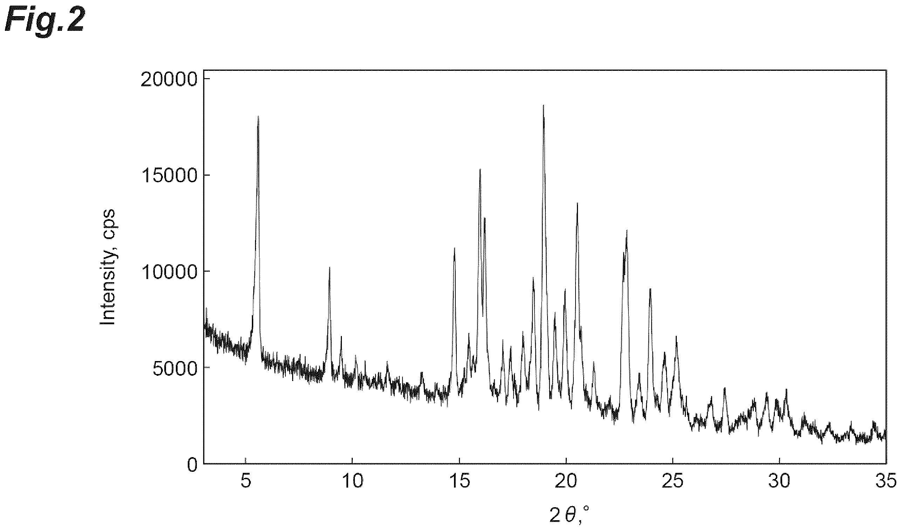

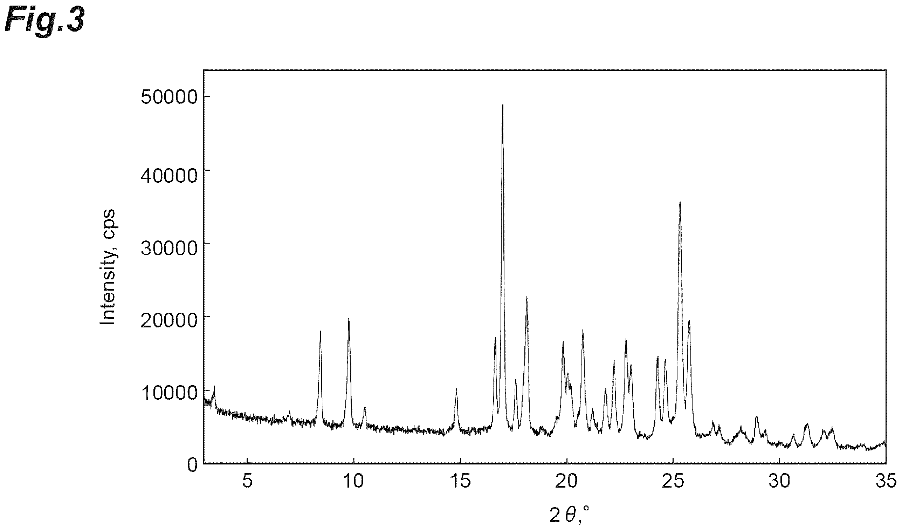

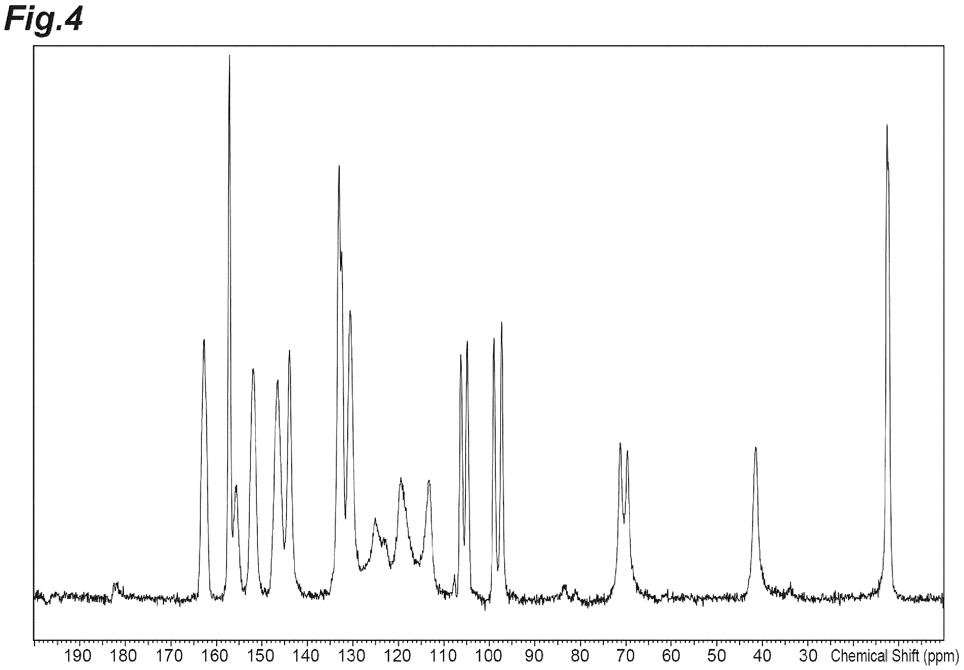

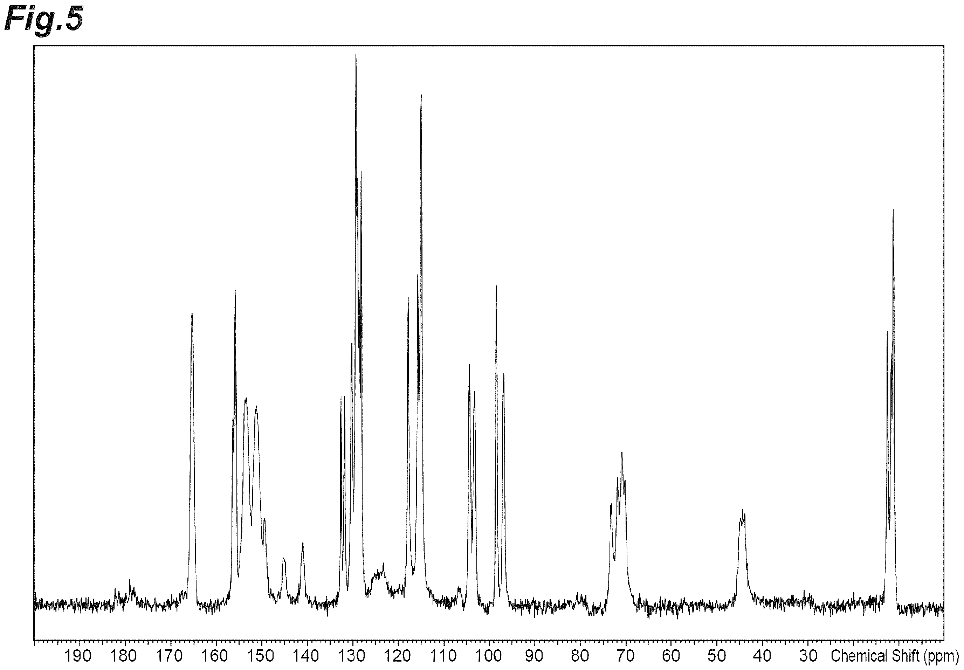

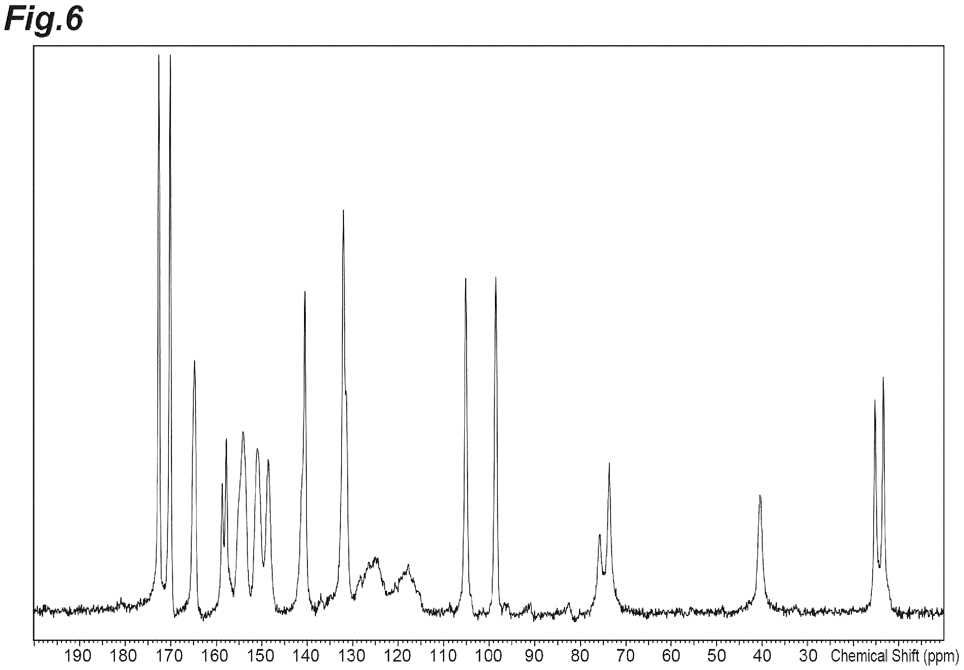

Specifically, the present specification relates to <1> to <31> in the following.
<1> A monophosphate or monomaleate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine-3-carboxamide represented by the following formula (I): 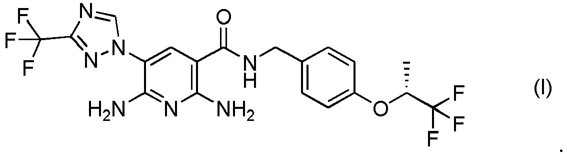
<2> A crystal of a monophosphate or monomaleate of 2,6-diamino-5-[3-(trifluoromethyl)-1H-1,2,4-triazol-1 -yl] -N-(4- {[(2R)-1,1,1 -trifluoropropan-2-yl]oxy}benzyl)pyridine- 3-carboxamide represented by the following formula (I): 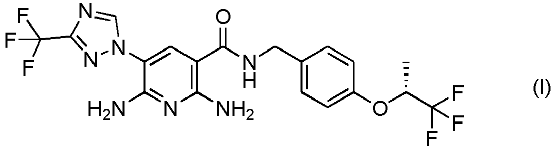
<3> An α crystal of a monophosphate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine- 3-carboxamide represented by the following formula (I): 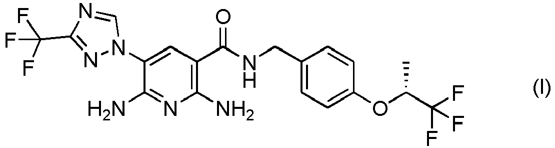 having a diffraction peak at a diffraction angle (2θ ± 0.2°) of 6.2° in a powder X-ray diffraction using CuKα as an X-ray source.
<4> An α crystal of a monophosphate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine- 3-carboxamide represented by the following formula (I): 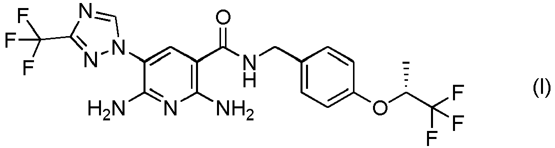 having diffraction peaks at diffraction angles (2θ ± 0.2°) of 6.2°, 12.5° and 14.3° in a powder X-ray diffraction using CuKα as an X-ray source.
<5> An α crystal of a monophosphate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine- 3-carboxamide represented by the following formula (I): 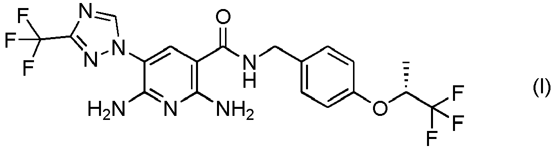 having diffraction peaks at diffraction angles (2θ ± 0.2°) of 6.2°, 11.1°, 12.5°, 14.3° and 24.9° in a powder X-ray diffraction using CuKα as an X-ray source.
<5-2> An α crystal of a monophosphate of 2,6-diamino-5-[3-(trifluoromethyl)-1H-1,2,4-triazol-1 -yl]-N-(4- {[(2R)-1,1,1 -trifhioropropan-2-yl]oxy}benzyl)pyridine-3-carboxamide represented by the following formula (I): 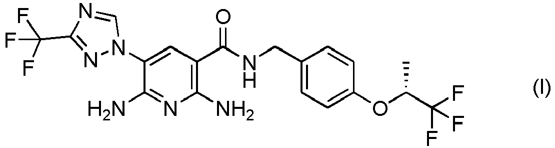 having diffraction peaks at diffraction angles (2θ ± 0.2°) of 6.2°, 11.1°, 12.5°, 14.3°, 16.7°, 19.7°, 21.5°, 22.4°, 24.9° and 28.7° in a powder X-ray diffraction using CuKα as an X-ray source.
<6> An α crystal of a monophosphate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine- 3-carboxamide represented by the following formula (I): 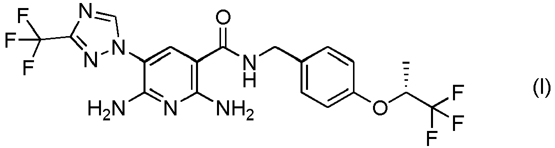 having a powder X-ray diffraction pattern substantially identical to a powder X-ray diffraction pattern shown in Figure 1 in a powder X-ray diffraction using CuKα as an X-ray source.
<7> An α crystal of a monophosphate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine- 3-carboxamide represented by the following formula (I): 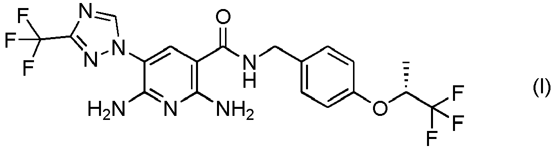 having a peak at a chemical shift (δ ± 0.5 ppm) of 157.2 ppm in a ¹³C solid state NMR spectrum with glycine as an external reference.
<8> An α crystal of a monophosphate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine- 3-carboxamide represented by the following formula (I): 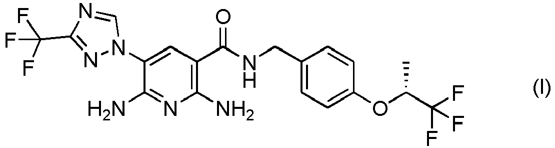 having peaks at chemical shifts (δ ± 0.5 ppm) of 41.4 ppm, 157.2 ppm and 162.7 ppm in a ¹³C solid state NMR spectrum with glycine as an external reference.
<9> An α crystal of a monophosphate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine- 3-carboxamide represented by the following formula (I): 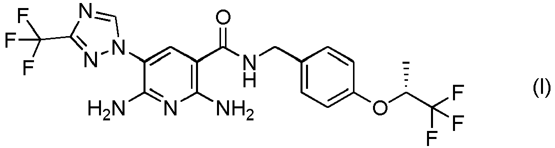 having peaks at chemical shifts (δ ± 0.5 ppm) of 41.4 ppm, 144.0 ppm, 146.5 ppm, 157.2 ppm and 162.7 ppm in a ¹³C solid state NMR spectrum with glycine as an external reference.
<9-2> An α crystal of a monophosphate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine- 3-carboxamide represented by the following formula (I): 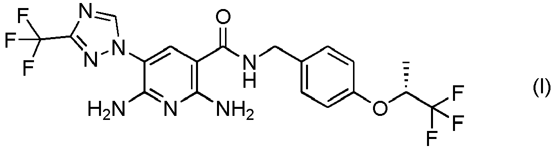 having peaks at chemical shifts (δ ± 0.5 ppm) of 41.4 ppm, 69.7 ppm, 106.3 ppm, 113.3 ppm, 119.0 ppm, 144.0 ppm, 146.5 ppm, 152.0 ppm, 157.2 ppm and 162.7 ppm in a ¹³C solid state NMR spectrum with glycine as an external reference.
<10> An α crystal of a monophosphate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine-3-carboxamide represented by the following formula (I): 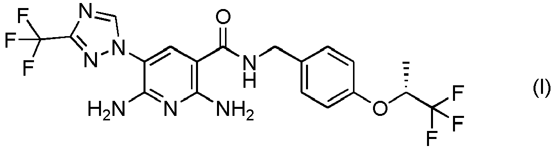 having a ¹³C solid state NMR spectrum substantially identical to a ¹³C solid state NMR spectrum shown in Figure 4 in a ¹³C solid state NMR spectrum with glycine as an external reference.
<11> A β crystal of a monophosphate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine-3-carboxamide represented by the following formula (I): 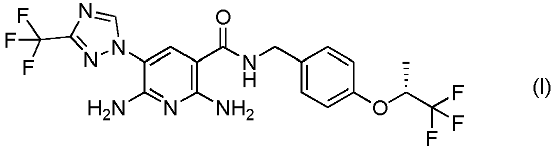 having a diffraction peak at a diffraction angle (2θ ± 0.2°) of 5.6° in a powder X-ray diffraction using CuKα as an X-ray source.
<12> A β crystal of a monophosphate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine-3-carboxamide represented by the following formula (I): 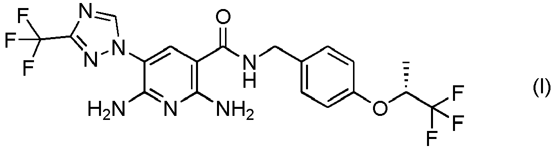 having diffraction peaks at diffraction angles (2θ ± 0.2°) of 5.6°, 9.0° and 16.0° in a powder X-ray diffraction using CuKα as an X-ray source.
<13> A β crystal of a monophosphate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine-3-carboxamide represented by the following formula (I): 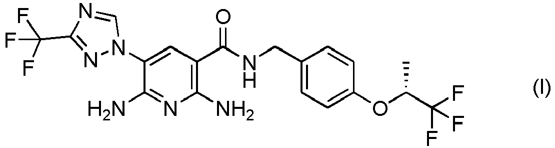 having diffraction peaks at diffraction angles (2θ ± 0.2°) of 5.6°, 9.0°, 16.0°, 22.8° and 23.9° in a powder X-ray diffraction using CuKα as an X-ray source.
<13-2> A β crystal of a monophosphate of 2,6-diamino-5-[3-(trifluoromethyl)-1H-1,2,4-triazol-1 -yl]-N-(4- {[(2R)-1,1,1 -trifhioropropan-2-yl]oxy}benzyl)pyridine-3-carboxamide represented by the following formula (I): 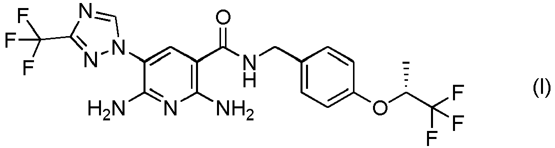 having diffraction peaks at diffraction angles (2θ ± 0.2°) of 5.6°, 9.0°, 16.0°, 18.5°, 19.5°, 19.9°, 22.8° and 23.9° in a powder X-ray diffraction using CuKα as an X-ray source.
<14> A β crystal of a monophosphate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine-3-carboxamide represented by the following formula (I): 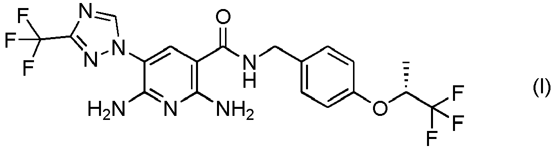 having a powder X-ray diffraction pattern substantially identical to a powder X-ray diffraction pattern shown in Figure 2 in a powder X-ray diffraction using CuKα as an X-ray source.
<15> A β crystal of a monophosphate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine-3-carboxamide represented by the following formula (I): 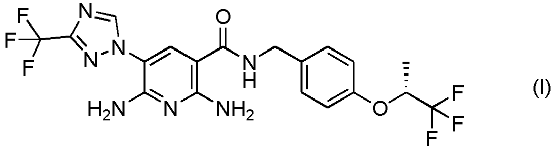 having a peak at a chemical shift (δ ± 0.5 ppm) of 129.3 ppm in a ¹³C solid state NMR spectrum with glycine as an external reference.
<16> A β crystal of a monophosphate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine-3-carboxamide represented by the following formula (I): 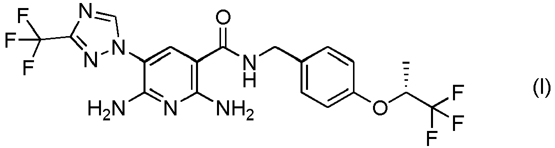 having peaks at chemical shifts (δ ± 0.5 ppm) of 11.2 ppm, 115.0 ppm and 129.3 ppm in a ¹³C solid state NMR spectrum with glycine as an external reference.
<17> A β crystal of a monophosphate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine-3-carboxamide represented by the following formula (I): 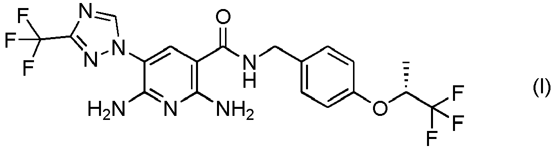 having peaks at chemical shifts (δ ± 0.5 ppm) of 11.2 ppm, 115.0 ppm, 117.9 ppm, 129.3 ppm and 165.5 ppm in a ¹³C solid state NMR spectrum with glycine as an external reference.
<17-2> A β crystal of a monophosphate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine-3-carboxamide represented by the following formula (I): 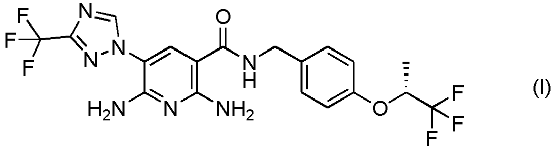 having peaks at chemical shifts (δ ± 0.5 ppm) of 11.2 ppm, 73.2 ppm, 103.2 ppm, 115.0 ppm, 117.9 ppm, 127.7 ppm, 129.3 ppm and 165.5 ppm in a ¹³C solid state NMR spectrum with glycine as an external reference.
<18> A β crystal of a monophosphate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine-3-carboxamide represented by the following formula (I): 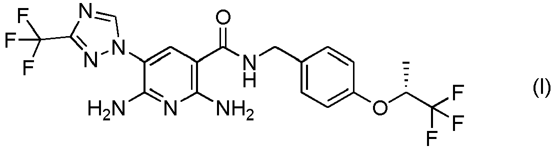 having a ¹³C solid state NMR spectrum substantially identical to a ¹³C solid state NMR spectrum shown in Figure 5 in a ¹³C solid state NMR spectrum with glycine as an external reference.
<19> A crystal of a monomaleate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine- 3-carboxamide represented by the following formula (I): 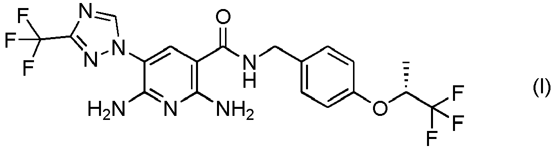 having a diffraction peak at a diffraction angle (2θ ± 0.2°) of 17.0° in a powder X-ray diffraction using CuKα as an X-ray source.
<20> A crystal of a monomaleate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine- 3-carboxamide represented by the following formula (I): 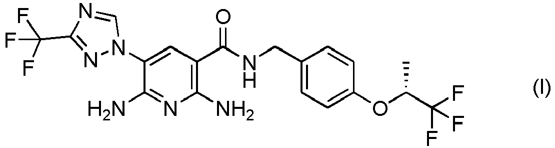 having diffraction peaks at diffraction angles (2θ ± 0.2°) of 17.0°, 18.2° and 25.3° in a powder X-ray diffraction using CuKα as an X-ray source.
<21> A crystal of a monomaleate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine- 3-carboxamide represented by the following formula (I): 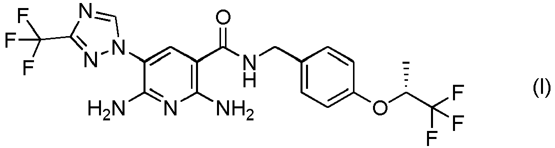 having diffraction peaks at diffraction angles (2θ ± 0.2°) of 8.5°, 9.9°, 17.0°, 18.2° and 25.3° in a powder X-ray diffraction using CuKα as an X-ray source.
<21-2> A crystal of a monomaleate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine- 3-carboxamide represented by the following formula (I): 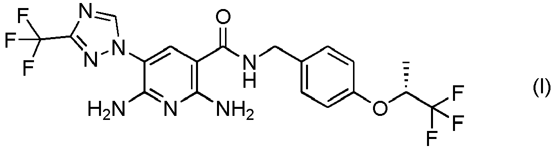 having diffraction peaks at diffraction angles (2θ ± 0.2°) of 8.5°, 9.9°, 17.0°, 18.2°, 19.8°, 20.8°, 22.2°, 22.8°, 24.3° and 25.3° in a powder X-ray diffraction using CuKα as an X-ray source.
<22> A crystal of a monomaleate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine- 3-carboxamide represented by the following formula (I): 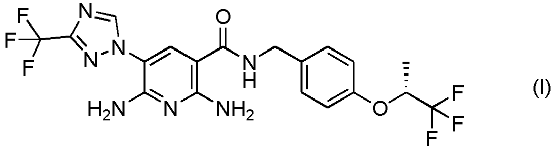 having a powder X-ray diffraction pattern substantially identical to a powder X-ray diffraction pattern shown in Figure 3 in a powder X-ray diffraction using CuKα as an X-ray source.
<23> A crystal of a monomaleate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine- 3-carboxamide represented by the following formula (I): 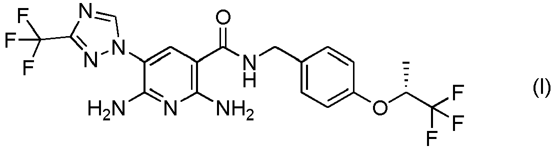 having a peak at a chemical shift (δ ± 0.5 ppm) of 172.6 ppm in a ¹³C solid state NMR spectrum with glycine as an external reference.
<24> A crystal of a monomaleate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine- 3-carboxamide represented by the following formula (I): 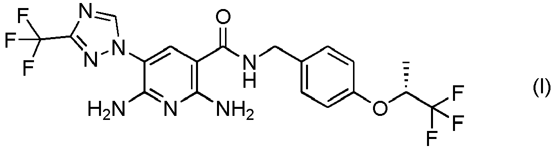 having peaks at chemical shifts (δ ± 0.5 ppm) of 132.0 ppm, 170.2 ppm and 172.6 ppm in a ¹³C solid state NMR spectrum with glycine as an external reference.
<25> A crystal of a monomaleate of 2,6-diamino-5-[3-(trifluoromethyl)-1H-1,2,4-triazol-1-yl]-N-(4-{[(2R)-1,1,1-trifhioropropan-2-yl]oxy}benzyl)pyridine- 3-carboxamide represented by the following formula (I): 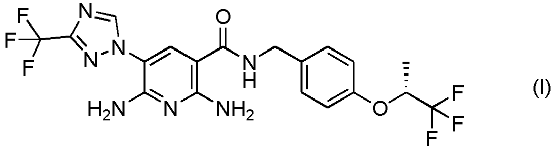 having peaks at chemical shifts (δ ± 0.5 ppm) of 98.5 ppm, 105.2 ppm, 132.0 ppm, 170.2 ppm and 172.6 ppm in a ¹³C solid state NMR spectrum with glycine as an external reference.
<25-2> A crystal of a monomaleate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine- 3-carboxamide represented by the following formula (I): 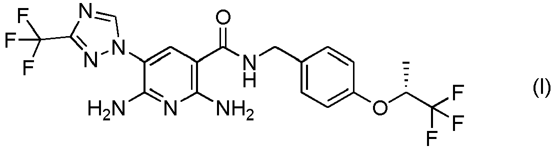 having peaks at chemical shifts (δ ± 0.5 ppm) of 13.4 ppm, 15.2 ppm, 40.5 ppm, 73.7 ppm, 98.5 ppm, 105.2 ppm, 132.0 ppm, 140.5 ppm, 164.8 ppm, 170.2 ppm and 172.6 ppm in a ¹³C solid state NMR spectrum with glycine as an external reference.
<26> A crystal of a monomaleate of 2,6-diamino-5-[3-(trifluoromethyl)-1H-1,2,4-triazol-1-yl]-N-(4-{[(2R)-1,1,1-trifhioropropan-2-yl]oxy}benzyl)pyridine- 3-carboxamide represented by the following formula 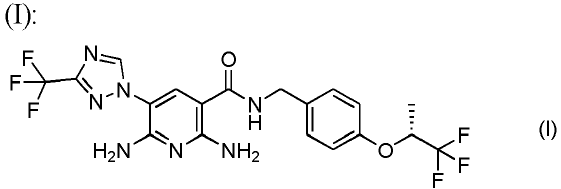 having a ¹³C solid state NMR spectrum substantially identical to a ¹³C solid state NMR spectrum shown in Figure 6 in a ¹³C solid state NMR spectrum with glycine as an external reference.
<27> A pharmaceutical composition comprising the salt according to <1> or the crystal according to any one of <2> to <26>.
<28> A prophylactic agent or therapeutic agent for malaria, comprising the salt according to <1> or the crystal according to any one of <2> to <26>.
<29> A method for preventing or treating malaria in a mammal, comprising administering an effective amount of the salt according to <1> or the crystal according to any one of <2> to <26> to the mammal.
<30> The salt according to <1> or the crystal according to any one of <2> to <26> for use in a method for preventing or treating malaria.
<31> Use of the salt according to <1> or the crystal according to any one of <2> to <26> for the manufacture of a pharmaceutical composition for preventing or treating malaria.

### Advantageous Effects of Invention

The prevent specification allows crystals of compound (I) with good physical properties to be provided, the crystals being expected to have a potential use as a drug substance for pharmaceuticals.

### Brief Description of Drawings

[Fig. 1] Figure 1 shows a powder X-ray diffraction pattern for an α crystal of a monophosphate of compound (I) obtained in Example 1. The abscissa shows diffraction angles (2θ), and the ordinate shows peak intensities.
[Fig. 2] Figure 2 shows a powder X-ray diffraction pattern for a crystal of a monophosphate of compound (I) in the β crystal form obtained in Example 2. The abscissa shows diffraction angles (2θ), and the ordinate shows peak intensities.
[Fig. 3] Figure 3 shows a powder X-ray diffraction pattern for a crystal of a monomaleate of compound (I) obtained in Example 3. The abscissa shows diffraction angles (2θ), and the ordinate shows peak intensities.
[Fig. 4] Figure 4 shows a ¹³C solid state NMR spectrum for an α crystal of a monophosphate of compound (I) obtained in Example 1. The abscissa shows chemical shifts (δ), and the ordinate shows peak intensities.
[Fig. 5] Figure 5 shows a ¹³C solid state NMR spectrum for a β crystal of a monophosphate of compound (I) obtained in Example 2. The abscissa shows chemical shifts (δ), and the ordinate shows peak intensities.
[Fig. 6] Figure 6 shows a ¹³C solid state NMR spectrum of a crystal of a monomaleate of compound (I) obtained in Example 3. The abscissa shows chemical shifts (δ), and the ordinate shows peak intensities.
[Fig. 7] Figure 7 shows a chart of the thermal analyses TG-DTA for an α crystal of a monohydrochloride of compound (I) obtained in Example 1. The abscissa shows temperatures, the left ordinate shows weight changes in TG, and the right ordinate shows heat flows in DTA.
[Fig. 8] Figure 8 shows a chart of the thermal analyses TG-DTA for a β crystal of a monophosphate of compound (I) obtained in Example 2. The abscissa shows temperatures, the left ordinate shows weight changes in TG, and the right ordinate shows heat flows in DTA.
[Fig. 9] Figure 9 shows a chart of the thermal analyses TG-DTA for a crystal of a monomaleate of compound (I) obtained in Example 3. The abscissa shows temperatures, the left ordinate shows weight changes in TG, and the right ordinate shows heat flows in DTA.

### Description of Embodiments

The salts of compound (I) of the present invention and crystals thereof, and production methods therefor will be hereinafter described in detail.

The term "salt" herein means a chemical consisting of compound (I), as a basic component, and an acid in a specific number of equivalents to compound (I).

Examples of "salt" to be used herein include salts with inorganic acid, salts with organic acid, and salts with acidic amino acid, and pharmaceutically acceptable salts are preferable among them.

Examples of salts with inorganic acid include salts with hydrochloric acid, hydrobromic acid, sulfuric acid, nitric acid, or phosphoric acid, and examples of salts with organic acid include salts with organic carboxylic acid such as acetic acid, succinic acid, fumaric acid, maleic acid, tartaric acid, malic acid, citric acid, lactic acid, stearic acid, and benzoic acid, and salts with organic sulfonic acid such as methanesulfonic acid (mesylic acid), ethanesulfonic acid, benzenesulfonic acid, and p-toluenesulfonic acid (tosylic acid); phosphoric acid and maleic acid are preferable among them.

Examples of salts with acidic amino acid include salts with aspartic acid or glutamic acid.

Each salt in the present specification may be an anhydride or a hydrate or solvate. The term hydrate or solvate refers to a solid that a molecule of compound (I) or a salt thereof and water molecules or solvent molecules are forming together, wherein the solid may be a crystal, and examples of solvent in solvates include ketone solvents such as acetone, 2-butanone, and cyclohexanone; ester solvents such as methyl acetate and ethyl acetate; ether solvents such as 1,2-dimethoxyethane and t-butyl methyl ether; alcoholic solvents such as methanol, ethanol, 1-propanol, and isopropanol; and polar solvents such as N-methyl-2-pyrrolidone, N,N-dimethylformamide, and dimethyl sulfoxide. The number of water molecules or solvent molecules per molecule of compound (I) or a salt thereof is not limited, and may be, for example, one or two.

The term "crystal" herein means a crystal of an anhydrate or hydrate of compound (I) or a salt thereof.

Preferable examples of crystals of a monophosphate and monomaleate of compound (I) in the present specification include:
an α crystal of a monophosphate of compound (I) having a diffraction peak at a diffraction angle (2θ ± 0.2°) of 6.2° in a powder X-ray diffraction using CuKα as an X-ray source;
an α crystal of a monophosphate of compound (I) having diffraction peaks at diffraction angles (2θ ± 0.2°) of 6.2°, 12.5° and 14.3° in a powder X-ray diffraction using CuKα as an X-ray source;
an α crystal of a monophosphate of compound (I) having diffraction peaks at diffraction angles (2θ ± 0.2°) of 6.2°, 11.1°, 12.5°, 14.3° and 24.9° in a powder X-ray diffraction using CuKα as an X-ray source;
an α crystal of a monophosphate of compound (I) having diffraction peaks at diffraction angles (2θ ± 0.2°) of 6.2°, 11.1°, 12.5°, 14.3°, 16.7°, 19.7°, 21.5°, 22.4°, 24.9° and 28.7° in a powder X-ray diffraction using CuKα as an X-ray source;
an α crystal of a monophosphate of compound (I) having a powder X-ray diffraction pattern substantially identical to a powder X-ray diffraction pattern shown in Figure 1 in a powder X-ray diffraction using CuKα as an X-ray source;
an α crystal of a monophosphate of compound (I) having a peak at a chemical shift (δ ± 0.5 ppm) of 157.2 ppm in a ¹³C solid state NMR spectrum with glycine as an external reference;
an α crystal of a monophosphate of compound (I) having peaks at chemical shifts (δ ± 0.5 ppm) of 41.4 ppm, 157.2 ppm and 162.7 ppm in a ¹³C solid state NMR spectrum with glycine as an external reference;
an α crystal of a monophosphate of compound (I) having peaks at chemical shifts (δ ± 0.5 ppm) of 41.4 ppm, 144.0 ppm, 146.5 ppm, 157.2 ppm and 162.7 ppm in a ¹³C solid state NMR spectrum with glycine as an external reference;
an α crystal of a monophosphate of compound (I) having peaks at chemical shifts (δ ± 0.5 ppm) of 41.4 ppm, 69.7 ppm, 106.3 ppm, 113.3 ppm, 119.0 ppm, 144.0 ppm, 146.5 ppm, 152.0 ppm, 157.2 ppm and 162.7 ppm in a ¹³C solid state NMR spectrum with glycine as an external reference;
an α crystal of a monophosphate of compound (I) having a ¹³C solid state NMR spectrum substantially identical to a ¹³C solid state NMR spectrum shown in Figure 4 in a ¹³C solid state NMR spectrum with glycine as an external reference;
a β crystal of a monophosphate of compound (I) having a diffraction angle (2θ ± 0.2°) of 5.6° in a powder X-ray diffraction using CuKα as an X-ray source;
a β crystal of a monophosphate of compound (I) having diffraction peaks at diffraction angles (2θ ± 0.2°) of 5.6°, 9.0° and 16.0° in a powder X-ray diffraction using CuKα as an X-ray source;
a β crystal of a monophosphate of compound (I) having diffraction peaks at diffraction angles (2θ ± 0.2°) of 5.6°, 9.0°, 16.0°, 22.8° and 23.9° in a powder X-ray diffraction using CuKα as an X-ray source;
a β crystal of a monophosphate of compound (I) having diffraction peaks at diffraction angles (2θ ± 0.2°) of 5.6°, 9.0°, 16.0°, 18.5°, 19.5°, 19.9°, 22.8° and 23.9° in a powder X-ray diffraction using CuKα as an X-ray source;
a β crystal of a monophosphate of compound (I) having a powder X-ray diffraction pattern substantially identical to a powder X-ray diffraction pattern shown in Figure 2 in a powder X-ray diffraction using CuKα as an X-ray source;
a β crystal of a monophosphate of compound (I) having a peak at a chemical shift (δ ± 0.5 ppm) of 129.3 ppm in a ¹³C solid state NMR spectrum with glycine as an external reference;
a β crystal of a monophosphate of compound (I) having peaks at chemical shifts (δ ± 0.5 ppm) of 11.2 ppm, 115.0 ppm and 129.3 ppm in a ¹³C solid state NMR spectrum with glycine as an external reference;
a β crystal of a monophosphate of compound (I) having peaks at chemical shifts (δ ± 0.5 ppm) of 11.2 ppm, 115.0 ppm, 117.9 ppm, 129.3 ppm and 165.5 ppm in a ¹³C solid state NMR spectrum with glycine as an external reference;
a β crystal of a monophosphate of compound (I) having peaks at chemical shifts (δ ± 0.5 ppm) of 11.2 ppm, 73.2 ppm, 103.2 ppm, 115.0 ppm, 117.9 ppm, 127.7 ppm, 129.3 ppm and 165.5 ppm in a ¹³C solid state NMR spectrum with glycine as an external reference;
a β crystal of a monophosphate of compound (I) having a ¹³C solid state NMR spectrum substantially identical to a ¹³C solid state NMR spectrum shown in Figure 5 in a ¹³C solid state NMR spectrum with glycine as an external reference;
a crystal of a monomaleate of compound (I) having a diffraction peak at a diffraction angle (2θ ± 0.2°) of 17.0° in a powder X-ray diffraction using CuKα as an X-ray source;
a crystal of a monomaleate of compound (I) having diffraction peaks at diffraction angles (2θ ± 0.2°) of 17.0°, 18.2° and 25.3° in a powder X-ray diffraction using CuKα as an X-ray source;
a crystal of a monomaleate of compound (I) having diffraction peaks at diffraction angles (2θ ± 0.2°) of 8.5°, 9.9°, 17.0°, 18.2° and 25.3° in a powder X-ray diffraction using CuKα as an X-ray source;
a crystal of a monomaleate of compound (I) having diffraction peaks at diffraction angles (2θ ± 0.2°) of 8.5°, 9.9°, 17.0°, 18.2°, 19.8°, 20.8°, 22.2°, 22.8°, 24.3° and 25.3° in a powder X-ray diffraction using CuKα as an X-ray source;
a crystal of a monomaleate of compound (I) having a powder X-ray diffraction pattern substantially identical to a powder X-ray diffraction pattern shown in Figure 3 in a powder X-ray diffraction using CuKα as an X-ray source;
a crystal of a monomaleate of compound (I) having a peak at a chemical shift (δ ± 0.5 ppm) of 172.6 ppm in a ¹³C solid state NMR spectrum with glycine as an external reference;
a crystal of a monomaleate of compound (I) having peaks at chemical shifts (δ ± 0.5 ppm) of 132.0 ppm, 170.2 ppm and 172.6 ppm in a ¹³C solid state NMR spectrum with glycine as an external reference;
a crystal of a monomaleate of compound (I) having peaks at chemical shifts (δ ± 0.5 ppm) of 98.5 ppm, 105.2 ppm, 132.0 ppm, 170.2 ppm and 172.6 ppm in a ¹³C solid state NMR spectrum with glycine as an external reference;
a crystal of a monomaleate of compound (I) having peaks at chemical shifts (δ ± 0.5 ppm) of 13.4 ppm, 15.2 ppm, 40.5 ppm, 73.7 ppm, 98.5 ppm, 105.2 ppm, 132.0 ppm, 140.5 ppm, 164.8 ppm, 170.2 ppm and 172.6 ppm in a ¹³C solid state NMR spectrum with glycine as an external reference; and
a crystal of a monomaleate of compound (I) having a ¹³C solid state NMR spectrum substantially identical to a ¹³C solid state NMR spectrum shown in Figure 6 in a ¹³C solid state NMR spectrum with glycine as an external reference.

Each of the diffraction peaks in powder X-ray diffraction and chemical shifts in ¹³C solid state NMR spectrum shown above is unique to the α crystal or β crystal of the monophosphate of compound (I) or the crystal of the monomaleate of compound (I), thus being a peak characteristic to the corresponding crystal.

Generally, errors in the range of ±0.2° may occur in diffraction angles (2θ) in powder X-ray diffraction, and hence it should be understood that each value of the diffraction angles shown above also covers numerical values in the range of about ±0.2° from the value. Accordingly, for a specific compound or a salt thereof, not only a crystal having peak diffraction angles exactly identical to those for the crystal of interest in powder X-ray diffraction but a crystal having peak diffraction angles each being within a margin of error of about ±0.2° from the corresponding peak diffraction angle is regarded as being identical to the crystal of interest, and included in the present invention.

For example, the phrase "having a diffraction peak at a diffraction angle (2θ ± 0.2°) of 6.2°" in the present specification means "having a diffraction peak at a diffraction angle (2θ) between 6.0° and 6.4°", and the same is applied to cases of other diffraction angles.

Generally, peak intensity or half width at a diffraction angle (2θ) in powder X-ray diffraction varies among different measurements even for identical crystal forms, because of the difference in measurement conditions or the variation of the sizes and shapes of the particles of powdery crystals used as measurement samples, and a constant peak intensity or half width is not necessarily exhibited in all cases. Therefore, even if difference in peak intensity or half width is found at the same diffraction angle (2θ) in comparing powder X-ray diffraction patterns, the difference does not indicate resulting from different crystal forms. Thus, a crystal having a powder X-ray diffraction pattern having diffraction peaks differing from those characteristics to a specific crystal of the present invention, as described, is regarded as being in a crystal form identical to that of the specific crystal of the present invention.

For example, the phrase "having a powder X-ray diffraction pattern substantially identical to a powder X-ray diffraction pattern shown in Figure 1" indicates that a certain powder X-ray diffraction pattern having certain characteristic diffraction peaks is a powder X-ray diffraction pattern identical to a powder X-ray diffraction pattern shown in Figure 1, not only if the certain powder X-ray diffraction pattern is exactly identical to the powder X-ray diffraction pattern shown in Figure 1, but also if the peak intensities or half widths are different from those in the powder X-ray diffraction pattern shown in Figure 1 or each characteristic diffraction peak agrees with the corresponding diffraction peak in the powder X-ray diffraction pattern shown in Figure 1 within an error range of diffraction angles of ±0.2°. Therefore, any crystal having such a powder X-ray diffraction pattern is regarded as a crystal identical to the corresponding crystal of the present invention.

The phrase "having a peak at a chemical shift (δ ± 0.5 ppm) of 157.2 ppm" in the present specification means "having a substantially equivalent peak to that at a chemical shift (δ ± 0.5 ppm) of 157.2 ppm in ¹³C solid state NMR spectral measurement with an external reference of glycine under common measurement conditions or conditions substantially identical to those in the present specification".

In determining whether to "have a substantially equivalent peak", generally, an error within a range of ±0.5 ppm may occur in chemical shifts, δ, in ¹³C solid state NMR spectrum, and hence it should be understood that each value of the chemical shifts shown above also covers numerical values in the range of about ±0.5 ppm from the value. Accordingly, not only a crystal having chemical shifts exactly identical to those for the crystal of interest in ¹³C solid state NMR spectrum but a crystal having chemical shifts each being within a margin of error of about ±0.5 ppm from the corresponding chemical shift is included in the present invention. Hence, in the present specification, for example, the phrase "having a peak at a chemical shift (δ ± 0.5 ppm) of 157.2 ppm" means "having a peak at a chemical shift (δ) between 156.7 ppm and 157.7 ppm", and the same is applied to cases of other chemical shifts in ¹³C solid state NMR spectrum.

The phrase "a crystal having a ¹³C solid state NMR spectrum substantially identical to a ¹³C solid state NMR spectrum shown in Figure 4" indicates that a crystal having a certain ¹³C solid state NMR spectrum having peaks at certain chemical shifts is a crystal having a ¹³C solid state NMR spectrum identical to a ¹³C solid state NMR spectrum shown in Figure 4, not only if the certain ¹³C solid state NMR spectrum is exactly identical to the ¹³C solid state NMR spectrum shown in Figure 4, but also if the peak intensities are different from those in the ¹³C solid state NMR spectrum shown in Figure 4 or each characteristic peak agrees with the corresponding peak in the ¹³C solid state NMR spectrum shown in Figure 4 within an error range of chemical shifts of about ±0.5 ppm. Therefore, any crystal having such a ¹³C solid state NMR spectrum is regarded as a crystal identical to the corresponding crystal of the present invention.

Production methods for salts, crystals, and the like of compound (I), each being an embodiment of the present invention, will be described in the following.

### Production Method for Compound (I)

Compound (I) may be one produced with a method well known to those skilled in the art. For example, compound (I) can be synthesized with a method described later in Reference Examples.

### Production Method for Salt of Compound (I)

Each of the salts of compound (I) according to the present specification can be obtained with a common method for producing a salt. Specifically, for example, each of the salts can be produced by suspending or dissolving compound (I) in a solvent with optional heating, followed by adding an acid to the resulting suspension or solution and stirring or leaving at room temperature or under cooling for several minutes to several days. Each salt of compound (I) can be obtained as a crystal or an amorphous form with the production method. The amorphous form can be obtained with the production method along with an additional operation such as freeze-drying, as necessary. Examples of the solvent applicable in this context include alcoholic solvents such as ethanol, 1-propanol, and isopropanol; acetonitrile; ketone solvents such as acetone and 2-butanone; ester solvents such as ethyl acetate; saturated hydrocarbon solvents such as hexane and heptane; ether solvents such as t-butyl methyl ether; and water. These solvents may be used singly, and two or more thereof may be used as a mixture.

### Production Method for Crystal of Compound (I) or Salt Thereof

Each of the crystals of compound (I) or a salt thereof can be produced with the above-described production method for compound (I) or production method for a salt thereof, and alternatively can be produced by dissolving compound (I) or a salt thereof in a solvent with heating and crystalizing it by cooling under stirring.

Compound (I) or a salt thereof to be used for the crystallization may be in any form, and may be in the form of a solvate or hydrate or an anhydride, and may be in an amorphous form or a crystalline form (including a form consisting of a plurality of crystalline polymorphs), and may be in the form of any mixture of them.

Examples of the solvent to be used for the crystallization include alcoholic solvents such as methanol, ethanol, isopropanol, and 1-propanol; acetonitrile; amide solvents such as N,N-dimethylformamide; ester solvents such as ethyl acetate; saturated hydrocarbon solvents such as hexane and heptane; ketone solvents such as acetone and 2-butanone; ether solvents such as t-butyl methyl ether; and water. These solvents may be used singly, and two or more thereof may be used as a mixture.

For the solvent, an appropriate amount of usage can be selected, on the assumption that the lower limit is the minimum amount that allows compound (I) or a salt thereof to dissolve through heating or allows the suspension to be stirred, and the upper limit is the maximum amount that does not cause significant reduction of the yield of a crystal.

In the crystallization, a seed crystal (such as a crystal of a desired salt of compound (I)) may be added or not. The temperature for addition of a seed crystal is not limited, and preferably 0 to 80°C.

For the temperature in dissolving compound (I) or a salt thereof by heating, an appropriate temperature that allows compound (I) or a salt thereof to dissolve can be selected according to the solvent, and the temperature is preferably in the range of 50°C to a temperature at which the recrystallization solvent begins to reflux, and more preferable 55 to 80°C.

In cooling in the crystallization, rapid cooling may result in a product containing crystals of different modes (polymorphs), and hence it is desirable to cool with appropriate adjustment of the cooling rate in view of the influence on the quality and grain size or the like of the crystal, and cooling is preferably performed, for example, at a rate of 5 to 40°C/hour. Cooling is more preferably performed, for example, at a rate of 5 to 25°C/hour.

An appropriate temperature can be selected for the final crystallization temperature in view of the yield and quality or the like of the crystal, and the final crystallization temperature is preferably -25 to 30°C.

The crystal resulting from the crystallization is separated through a common filtration process, the crystal separated through filtration is washed with a solvent, as necessary, and the resultant is further dried, giving the target crystal. Any of the solvents shown for the crystallization solvent can be used as the solvent for washing the crystal. Preferable examples thereof include ethanol, acetone, 2-butanone, ethyl acetate, diethyl ether, t-butyl methyl ether, and hexane. These solvents may be used singly, and two or more thereof may be used as a mixture.

The crystal separated through the filtration process can be appropriately dried by leaving in the atmosphere or under nitrogen gas flow, or by heating.

For the drying time, an appropriate time required for the residual solvent to reduce to a predetermined amount can be selected according to the production volume, the drier, the drying temperature, and others. Drying can be performed under air flow or under reduced pressure. An appropriate degree of vacuum can be selected according to the production volume, the drier, the drying temperature, and others. After drying, the resulting crystal can be left in the atmosphere, as necessary.

Salts of Compound (I) and crystals thereof obtained with the production methods described above each have a potential use as a prophylactic agent and/or therapeutic agent for malaria, as demonstrated by activity data shown later in Pharmacological Test Example.

### [Pharmaceutical Composition]

Another embodiment of the present invention is a pharmaceutical composition comprising a salt of compound (I) or a crystal thereof; and a pharmaceutically acceptable additive. The pharmaceutical composition can be produced by mixing a pharmaceutically acceptable additive well with a salt of compound (I) or a crystal thereof. The pharmaceutical composition according to the present invention can be produced with a known method such as a method described in General Rules for Preparations, The Japanese Pharmacopeia Seventeenth Edition. The pharmaceutical composition according to the present embodiment can be administered to a patient in a proper manner according to the dosage form.

The pharmaceutical composition can be orally administered in the form of a solid formulation such as a tablet, a granule, a fine granule, a powder, and a capsule, or a liquid formulation such as a solution, a jelly, and a syrup. The pharmaceutical composition may be parenterally administered, for example, in the form of an injection, a suppository, an ointment, or a cataplasm.

In preparing a solid formulation, a diluent and, as necessary, a binder, a disintegrant, a lubricant, a coloring agent, a corrigent, and so on are added as additives to a salt of compound (I) or a crystal thereof, and the resultant can be then formulated, for example, into a tablet, a granule, a fine granule, a powder, or a capsule by using a conventional method. The additives can be used in combination for formulation. The tablet, granule, or the like may be coated, as necessary.

Examples of the diluent include lactose, white soft sugar, glucose, cornstarch, mannitol, sorbitol, starch, pregelatinized starch, dextrin, microcrystalline cellulose, and calcium hydrogen phosphate.

Examples of the binder include methylcellulose, ethylcellulose, gum Arabic, hydroxypropylmethylcellulose, and hydroxypropylcellulose.

Examples of the disintegrant include low-substituted hydroxypropylcellulose, carboxymethylcellulose, carboxymethylcellulose calcium, croscarmellose sodium, sodium carboxymethyl starch, and crospovidone.

Examples of the lubricant include talc, silica, magnesium stearate, calcium stearate, sodium stearyl fumarate, and polyethylene glycol.

Examples of the coloring agent include red ferric oxide, yellow ferric oxide, carmine, β-carotene, titanium oxide, riboflavin sodium phosphate, yellow aluminum lake, and cochineal.

Examples of the corrigent include cocoa powder, ascorbic acid, tartaric acid, Mentha oil, borneol, and powdered cinnamon bark.

In preparing an injection, a pH adjuster, a buffer, a suspending agent, a solubilizer, a stabilizer, an isotonic agent, a preservative, and so on are added, as necessary, to a main drug, and the resultant can be formulated into an intravenous, subcutaneous, or intramuscular injection, or an intravenous infusion drip by using a conventional method. At that time, the injection can be prepared as a freeze-dried product, as necessary, by using a conventional method.

Examples of the pH adjuster and buffer include hydrochloric acid, sodium carbonate, sodium hydrogen carbonate, citric acid, sodium citrate, sodium dihydrogen citrate, glycine, phosphoric acid, sodium dihydrogen phosphate, sodium monohydrogen phosphate, sodium hydroxide, acetic acid, sodium acetate, and meglumine.

Examples of the suspending agent include sodium alginate, sucrose fatty acid ester, Polysorbate 80, gum Arabic, powdered tragacanth, and polyoxyethylene sorbitan monolaurate.

Examples of the solubilizer include ethoxylated hydrogenated castor oil, Polysorbate 80, nicotinamide, polyoxyethylene sorbitan monolaurate, glycerin fatty acid ester, polyethylene glycol, polypropylene glycol, benzyl benzoate, ethanol, and triethanolamine.

Examples of the stabilizer include sodium sulfite and sodium metasulfite.

Examples of the isotonic agent include glucose, mannitol, and sorbitol.

Examples of the preservative include methyl paraoxybenzoate, ethyl paraoxybenzoate, sorbic acid, phenol, cresol, and chlorocresol.

The dose of compound (I) according to the present invention depends on the degree of symptoms, age, sex, body weight, mode of administration, the type of the salt, the specific disease type, and others, and typically a dose of approximately 30 µg to 10 g, preferably of 100 µg to 5 g, more preferably of 100 µg to 1 g in the case of oral administration or a dose of approximately 30 µg to 1 g, preferably of 100 µg to 500 mg, more preferably of 100 µg to 300 mg in the case of administration by injection is administered once or separately in several portions per day for an adult.

### Examples

The crystals of compound (I) of the present invention can be produced, for example, with methods described in Examples shown below, and effects exerted by the compound can be confirmed with methods described later in Test Example shown below However, those methods are merely examples and the present invention is never limited by the specific examples shown below in any case, and modification may be made without departing from the scope of the present invention.

The term "room temperature" in Examples and Reference Examples in the following refers to approximately 10°C to approximately 35°C in normal situations. % indicates percent by weight unless otherwise stated.

### Reference Examples

The chemical names of the compounds of Examples were determined on the basis of the chemical structures presented by "E-Notebook 2014" version 13 (PerkinElmer Co., Ltd.). Pre-packed silica gel cartridges on a Biotage (R) Isolera Four (R) were used for silica gel chromatography.

Chemical shifts in ¹H NMR spectra were calculated with reference to tetramethylsilane (ppm = 0.00) as an internal standard substance, and the following abbreviations or combinations of them were used: br = broad signal, s = singlet, d = doublet, t = triplet, m = multiplet. ¹H-NMR was measured by using a Bruker AVIII (600 MHz).

Optical purities in ee were calculated by using a Shimadzu Chiral HPLC.

A Waters MDAP system equipped with a Waters SQD mass was used for purification by HPLC.

Further, the following abbreviations were used:
app: apparent
DCM: Dichloromethane
DIPEA: N,N-Diisopropylethylamine
DMF-DMA: N,N-Dimethylformamide dimethyl acetal
Dppf: 1,1'-Ferrocenediyl-bis(diphenylphosphine)
EDC.HCl: N-(3-Dimethylaminopropyl)-N-ethylcarbodiimide hydrochloride
HOBT: 1-Hydroxybenzotriazole
HPLC: High pressure liquid chromatography
IMS: Industrial Methylated Spirit
LCMS: Liquid chromatography-Mass Spectroscopy
MDAP: Mass Directed Autopurification
2-MeTHF: 2-Methyl Tetrahydrofuran
TBME: tert-Butyl methyl ether
NBS: N-Bromosuccinimide
NIS: N-Iodosuccinimide
SQD: Single quadrupole detection
TBTU: O-(Benzotriazol-1-yl)-N,N,N',N'-tetramethyluronium tetrafluoroborate
THF: Tetrahydrofuran
wherein the abbreviations used herein are well known and common to those skilled in the art.

Compound (I) can be produced, for example, with any method described in Reference Examples shown below, and the effects can be confirmed with methods described in Test Example shown below. However, those methods are merely examples and the present invention is never limited by the specific examples shown below in any case, and modification may be made without departing from the scope of the present invention.

### Reference Example 1

### Synthesis of 2,6-diamino-5-[3-(trifluoromethyl)-1H-1,2,4-triazol-1-yl]-N-(4-{[(2R)-1,1,1-trifluoropropan-2-yl]oxy}benzyl)pyridine-3-carboxamide (compound (I))

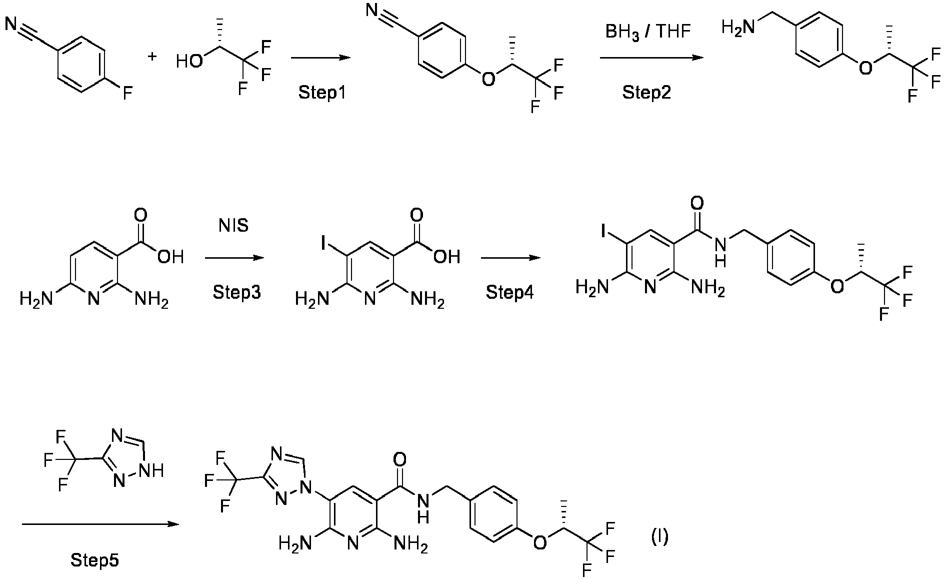

### Step 1: Synthesis of (R)-4-((1,1,1-trifluoropropan-2-yl)oxy)benzonitrile

To a suspension of potassium tert-butoxide (3.47 g, 30.9 mmol) in THF (35 mL), (R)-1,1,1-trifluoropropan-2-ol (2.00 mL, 21.7 mmol) was added with stirring under nitrogen gas flow at 0°C. After 10 min, a solution of 4-fluorobenzonitrile (2.5 g, 20.6 mmol) in THF (35 mL) was added dropwise, and the resultant was stirred for 1 hour and the temperature was then returned to room temperature. The reaction mixture was subjected to liquid separation with EtOAc/water, and the organic layer was washed with brine and then dried over Na₂SO₄. The solvent was distilled off with an evaporator, and the resulting residue (5.4 g) was purified by column chromatography (0-50% EtOAc/c-Hex, 50 g Silica-gel) to afford the title compound (4.27 g).
¹H-NMR(600MHz, CDCl₃) δ ppm 1.54(d, J=6.4Hz, 3H) 4.74(t, J=6.1Hz, 1H) 7.02(d, J=8.8Hz, 2H) 7.63(d, J=8.8Hz, 2H)

### Step 2: Synthesis of (R)-(4-((1,1,1-trifluoropropan-2-yl)oxy)phenyl)methanamine

To a solution of (R)-4-((1,1,1-trifluoropropan-2-yl)oxy)benzonitrile (4.1 g, 19.1 mmol) in THF (39 mL), 1 M solution of borane-tetrahydrofuran complex (38.1 mL, 38.1 mmol) in THF was added dropwise with stirring at 0°C. The temperature of the reaction mixture was returned from 0° to room temperature over 1 hour, and the reaction mixture was further heated at 65°C. After cooling to room temperature, 2 M HCl (39.0 mL, 78.0 mmol) was added dropwise, and the mixture was stirred with heating at 65°C for 2 hours and further at 100°C for 2 hours. The reaction mixture was cooled and then diluted with MeOH, and loaded in an SCX-2 cartridge. After washing with MeOH, the product was eluted with 2 M NH₃/MeOH. The eluates were collected, and the solvent was distilled off with an evaporator to afford the title compound (3.76 g).
1H NMR (600MHz, CDCl₃) δ ppm 1.49(d, J=6.6Hz, 3H) 3.83(s, 2H) 4.61(t, J=6.3Hz, 1H) 6.93(d, J=8.6Hz, 2H) 7.24-7.27(m, 2H)

### Step 3: Synthesis of 2,6-diamino-5-iodonicotinic acid

To a suspension of 2,6-diaminonicotinic acid (1.01 g, 6.60 mmol) in DMF (20 mL), NIS (1.63 g, 7.24 mmol) was added in small portions over 5 minutes. The starting raw material had been crushed into small pieces by heating and ultrasonication before the use. The reaction mixture was diluted with 1M NaOH, and subjected to liquid separation with EtOAc. The aqueous layer was acidified to pH 6 with concentrated hydrochloric acid. The precipitate was collected by filtration, and washed with water and EtOAc. The resultant was dried under reduced pressure to afford the title compound (1.28 g).
1H NMR (600MHz, DMSO-d₆) δ ppm 6.35(br s, 2H) 6.97(br s, 2H) 8.01(s, 1H) 12.15(br s, 1H)

### Step 4: Synthesis of (R)-2,6-diamino-5-iodo-N-(4-((1,1,1-trifluoropropan-2-yl)oxy)benzyl)nicotinamide

To a solution of 2,6-diamino-5-iodonicotinic acid (1 g, 3.58 mmol) in DMSO (10 mL), HOBT·H₂O (0.659 g, 4.30 mmol) and EDC·HCl (0.824 g, 4.30 mmol) were added with stirring. After 5 minutes, a solution of (R)-(4-((1,1,1-trifluoropropan-2-yl)oxy)phenyl)methanamine (0.825 g, 3.76 mmol) and triethylamine (2.00 mL, 14.3 mmol) in DMSO (10 mL) was added dropwise, and the resultant was stirred at room temperature for 20 hours. The reaction mixture was subjected to liquid separation with EtOAc/water, and the organic layer was washed with brine and then dried over Na₂SO₄. The solvent was distilled off with an evaporator, and the resulting residue (2.15 g) was purified by column chromatography (20-80 % EtOAc/c-Hex, 25 g KP-Sil) to afford the title compound (1.08 g).
1H NMR (600MHz, DMSO-d₆) δ ppm 1.39(d, J=6.4Hz, 3H) 4.30(d, J=5.7Hz, 2H) 5.16 (app spt, J=6.5Hz, 1H) 6.13(br s, 2H) 7.02(d, J=8.6Hz, 2H) 7.07(br s, 2H) 7.23(d, J=8.4Hz, 2H) 8.11(s, 1H) 8.51(t, J=5.8Hz, 1H)

### Step 5: Synthesis of 2,6-diamino-5-[3-(trifluoromethyl)-1H-1,2,4-triazol-1-yl]-N-(4-{[(2R)-1,1,1-trifluoropropan-2-yl]oxy}benzyl)pyridine-3-carboxamide (compound (I))

A solution of (R)-2,6-diamino-5-iodo-N-(4-((1,1,1-trifluoropropan-2-yl)oxy)benzyl)nicotinamide (120 mg, 0.25 mmol), 3-(trifluoromethyl)-1H-1,2,4-triazole (47.9 mg, 0.35 mmol), copper(I) iodide (52.3 mg, 0.275 mmol), N,N'-dimethylcyclohexane-1,2-diamine (0.043 mL, 0.275 mmol), and potassium phosphate (106 mg, 0.50 mmol) in DMF (2 mL) was subjected to gas replacement with nitrogen, and heated at 120°C for 18 hours in a sealed tube. The reaction mixture was diluted with MeOH, loaded in an SCX-2, and washed with MeOH. Subsequently, elution was performed with 2 M NH₃/MeOH/DCM to afford an oily substance (0.14 g). This was purified by column chromatography (20-100% EtOAc/c-Hex, KP-Sil) to afford the title compound (21 mg).
1H NMR (600MHz, DMSO-d6) δ ppm 1.38(d, J=6.4Hz, 3H) 4.31(d, J=5.7Hz, 2H) 5.15 (dt, J=12.8, 6.4Hz, 1H) 6.39(s, 2H) 7.01(d, J=8.4Hz, 2H) 7.23(d, J=8.4Hz, 2H) 7.42(br s, 2H) 7.92(s, 1H) 8.41(br t, J=5.8Hz, 1H) 8.97(s, 1H)

### Reference Example 2

### Synthesis of 2,6-diamino-5-[3-(trifluoromethyl)-1H-1,2,4-triazol-1-yl]-N-(4-{[(2R)-1,1,1-trifluoropropan-2-yl]oxy}benzyl)pyridine-3-carboxamide (compound (I))

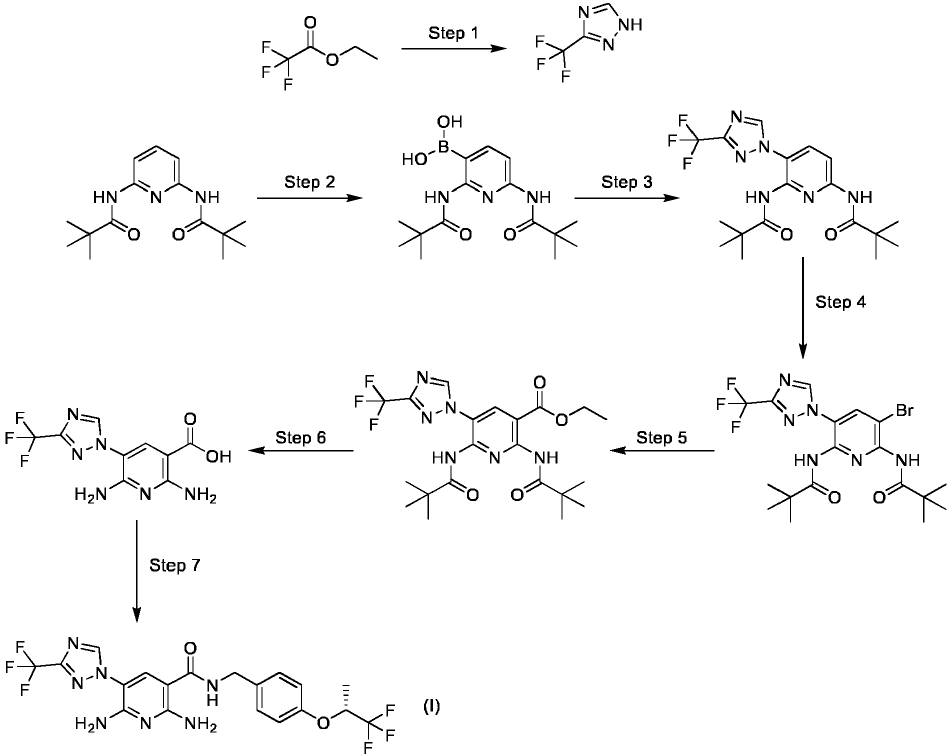

### Step 1: Synthesis of 3-(trifluoromethyl)-1H-1,2,4-triazole

To a solution of hydrazine monohydrate (100 mL, 2052 mmol) in EtOH (2.6 L), ethyl trifluoroacetate (257 mL, 2161 mmol) (CAS 383-63-1, Fluorochem Ltd.) was added dropwise with stirring at 10°C, and the reaction mixture was stirred at room temperature for 16 hours. Iminoformamide acetate (247 g, 2377 mmol) was added, and the reaction mixture was heated to reflux for 9 hours. AcOH (148 mL, 2585 mmol) was added, and EtOH was then distilled off with an evaporator. The resulting solution was basified with saturated aqueous sodium hydrogen carbonate solution, and then subjected to extraction with EtOAc (3 × 500 mL). The collected organic layer was dried over Na₂SO₄ and then filtered through a filter, and the solvent was distilled off with an evaporator. The resulting crude product was triturated with n-heptane, collected by filtration through a filter, and then washed with n-heptane (100 mL). The resultant was dried under reduced pressure to afford the title compound (216 g).
LCMS: m/z 138 [M+H]⁺. ¹H NMR (600 MHz, DMSO-cf) δ ppm 8.84 (s, 1 H) 13.99 (br s, 1 H).

### Step 2: Synthesis of (2,6-dipivalamidepyridin-3-yl)boronic acid

To a solution of N,N'-(pyridine-2,6-diyl)bis(2,2-dimethylpropanamide) (80 g, 288 mmol) in THF (1 L), n-BuLi (2.5 M hexane solution, 400 mL, 1000 mmol) was added dropwise with stirring under nitrogen gas flow at -70°C. The temperature of the reaction mixture was increased to 0°C, the reaction mixture was stirred for 16 hours and then cooled to -60°C, and triisopropyl borate (233 mL, 1009 mmol) was added dropwise. The temperature of the mixture was increased to room temperature, the mixture was stirred for 90 minutes and then cooled to 0°C, and saturated aqueous NH₄Cl solution (600 mL) was added dropwise. The mixture was separated by liquid separation with water (600 mL) and 2-MeTHF (200 mL). The aqueous layer was further subjected to extraction with 2-MeTHF (2 × 1L), the collected organic layer was washed with saturated aqueous NH₄Cl solution (2 × 500 mL), then dried over Na₂SO₄, and thereafter filtered through a filter, and the solvent was distilled off with an evaporator. The resulting residue was stirred in toluene (1 L) at room temperature for 16 hours. The resulting precipitate was collected by filtration, then washed with cooled toluene (200 mL), and thereafter dried under reduced pressure to afford the title compound (83.2 g).
LCMS: m/z 322 [M+H]⁺. ¹H NMR (600 MHz, DMSO-d6) δ ppm 1.17 (s, 9 H) 1.23 (s, 9 H) 7.69 (d, J=7.89 Hz, 1 H) 7.92 (br d, J=7.89 Hz, 1 H) 9.17 (s, 1 H) 11.43 (br s, 1 H).

### Step 3: Synthesis of N,N-(3-(3-(trifluoromethyl)-1H-1,2,4-triazol-1-yl)pyridine-2,6-diyl)bis(2,2-dimethylpropanamide)

To a solution of (2,6-dipivalamidepyridin-3-yl)boronic acid (30 g, 93.4 mmol) in DMF (600 mL), (trifluoromethyl)-1H-1,2,4-triazole (19.2 g, 140 mmol), copper(II) acetate (1.70 g, 9.34 mmol), and pyridine (18.9 mL, 234 mmol) were added in small portions with stirring. With use of a three-necked flask for reaction, air that had been passed through anhydrous CaCl₂ was introduced into the reaction mixture under moderately reduced pressure via a sparging tube. The reaction mixture was heated at 60°C for 22 hours and then cooled, and poured into iced water (2 L). After 30 minutes, the precipitate was collected by filtration, washed with water (200 mL) and n-heptane (200 mL), and then dried under reduced pressure to afford the title compound (32.0 g). LCMS: m/z 413 [M+H]⁺. ¹H NMR (600 MHz, DMSO-d6) δ ppm 1.03 (s, 9 H) 1.24-1.28 (m, 10 H) 8.11 (d, J=2.75 Hz, 2 H) 9.05 (s, 1 H) 9.88 (s, 1 H) 10.16 (s, 1 H).

### Step 4: Synthesis of N,N'-(3-bromo-5-(3-(trifluoromethyl)-1H-1,2,4-triazol-1-yl)pyridine-2,6-diyl)bis(2,2-dimethylpropanamide)

To a solution of N,N-(3-(3-(trifluoromethyl)-1H-1,2,4-triazol-1-yl)pyridine-2,6-diyl)bis(2,2-dimethylpropanamide) (76.1 g, 184 mmol) in DMF (1 L), NBS (34.5 g, 194 mmol) was added with stirring at room temperature. The reaction mixture was stirred at 70°C for 40 minutes, then cooled to 22°C, and poured into iced water (3 L) with stirring. After stirring at room temperature for 30 minutes, the precipitate was collected by filtration. The resulting solid was subjected to liquid separation with EtOAc (500 mL) and water (500 mL), and the separated organic layer was washed with brine (2 × 300 mL). After drying over MgSO₄, the solvent was concentrated with an evaporator. The resulting residue was triturated with n-heptane, and solids collected were dried under reduced pressure to afford the title compound (84.3 g).
LCMS: m/z 491/493 [M+H]⁺. ¹H NMR (600 MHz, DMSO-cf) δ ppm 1.04 (s, 9 H) 1.25 (s, 8 H) 8.59 (s, 1 H) 9.23 (s, 1 H) 9.87-9.93 (m, 1 H) 10.04 (s, 1 H) 10.28 (s, 1 H).

### Step 5: Synthesis of ethyl 2,6-dipivalamide-5-(3-(trifluoromethyl)-1H-1,2,4-triazol-1-yl)nicotinic acid

A solution of EtOH (152 mL), toluene (1.1 L), and Et₃N (443 mL, 3176 mmol) was subjected to gas replacement by passing nitrogen therethrough for 1 hour. To this solution, N,N-(3-bromo-5-(3-(trifluoromethyl)-1H-1,2,4-triazol-1-yl)pyridine-2,6-diyl)bis(2,2-dimethylpropanamide) (85.0 g, 173 mmol) and PdCl₂(dppf).DCM (28.3 g, 34.6 mmol) were added. CO gas (5 L) was introduced into the mixture, and the mixture was heated under CO gas flow at 80°C for 18 hours. The reaction mixture was cooled, Celite (R) (180 g) was then added, and the resultant was stirred at room temperature for 30 minutes. The mixture was flowed with EtOAc (2 L) through a silica pad. The liquid flowed out was distilled with an evaporator to afford the title compound (115.8 g).
LCMS: m/z 485 [M+H]⁺. ¹H NMR (600 MHz, DMSO-d6) δ ppm 1.05 (s, 9 H) 1.24 (s, 9 H) 1.26 -1.31 (m, 3 H) 4.21 - 4.28 (m, 2 H) 8.45 (s, 1 H) 9.17 (s, 1 H) 10.59 (s, 1 H).

### Step 6: Synthesis of 2,6-diamino-5-(3-(trifluoromethyl)-1H-1,2,4-triazol-1-yl)nicotinic acid ethyl

To a solution of 2,6-dipivalamide-5-(3-(trifluoromethyl)-1H-1,2,4-triazol-1-yl)nicotinic acid (84 g, 174 mmol) in IMS (432 mL), 4 M aqueous NaOH solution (867 mL, 3467 mmol) was added with stirring at 80°C. The reaction mixture was heated to reflux for 5 hours, then cooled, diluted with iced water (1.3 L) with stirring, and filtered through a filter, and thereafter IMS was distilled off with an evaporator. The aqueous solution was washed with TBME (2 × 1.5 L), then acidified with concentrated hydrochloric acid (50 mL) and 2 M HCl (300 mL) to pH 5, and left to stand at room temperature for 18 hours, resulting in formation of a precipitate. The solid was collected by filtration through a filter, then washed with water (2 × 300 mL) and Et₂O (2 × 300 mL), and thereafter dried under reduced pressure to afford the title compound (51 g).
LCMS: m/z 289 [M+H]⁺. ¹H NMR (600 MHz, DMSO-d₆) δ ppm 6.60 (br s, 2 H) 7.45 (br s, 2 H) 7.79 (s, 1 H) 8.97 (s, 1 H) 12.24 (br s, 1 H).

### Step 7: Synthesis of 2,6-diamino-5-[3-(trifluoromethyl)-1H-1,2,4-triazol-1-yl]-N-(4- {[(2R)-1,1,1-trifluoropropan-2-yl]oxy}benzyl)pyridine-3-carboxamide (compound (I))

To a solution of 2,6-diamino-5-(3-(trifluoromethyl)-1H-1,2,4-triazol-1-yl)nicotinic acid (44.6 g, 155 mmol) and TBTU (52.2 g, 163 mmol) in DMF (650 mL), DIPEA (108 mL, 619 mmol) was added with stirring at room temperature. The mixture was stirred at room temperature for 15 minutes, (R)-(4-((1,1,1-trifluoropropan-2-yl)oxy)phenyl)methanamine hydrochloride (41.5 g, 163 mmol) was then added, and the mixture was further stirred at room temperature for 1 hour. The reaction mixture was diluted with EtOAc (1.2 L) and semi-saturated brine (1 L). The organic layer was washed with water (500 mL), then diluted with brine (500 mL), and filtered through Celite (R) with EtOAc (1 L). The organic layer was washed with brine (500 mL), then dried over MgSO₄, and the solvent was distilled off with an evaporator. The residue was suspended in 20% EtOH/n-heptane (800 mL), and the resultant was heated to reflux for 30 minutes. After cooling, the solid was collected by filtration through a filter, washed with 20% EtOH/n-heptane (2 × 350 mL) and n-pentane (2 × 400 mL), and then dissolved in EtOAc (500 mL). The operations were repeated three times, and the collected product was eluted with EtOAc (7 L) through KP-NH silica (500 g). The solvent of the eluate was distilled off, and the resulting residue was triturated with n-pentane, and then dried under reduced pressure to afford the title compound (50 g). LCMS: m/z 490 [M+H]⁺. ¹H NMR (600 MHz, DMSO-d₆) δ ppm 1.39 (d, J=6.24 Hz, 3 H) 4.31 (d, J=5.69 Hz, 2 H) 5.15 (app spt, J=6.42 Hz, 1 H) 6.39 (s, 2 H) 7.01 (d, J=8.25 Hz, 2 H) 7.23 (d, J=8.44 Hz, 2 H) 7.42 (br s, 2 H) 7.92 (s, 1 H) 8.41 (br t, J=5.69 Hz, 1 H) 8.97 (s, 1 H). Chiral HPLC: Rₜ 20.3 mins (minor), 22.9 mins (major), 96.6% ee.

### Example 1

### Preparation of α-Type Crystal of Monophosphate of Compound (I)

To a solution of compound (I) (5 g) in acetonitrile (150 mL), a solution of phosphoric acid (1.20 g) in acetonitrile (50 mL) was added dropwise at room temperature over 10 minutes. After the dropwise addition, the resultant was stirred at room temperature overnight, and a solid precipitated was then collected by filtration, washed with acetonitrile (20 mL), and then dried under air flow to afford the title crystal (5.68 g).

Powder X-ray diffraction peaks (transmission method, 2θ ± 0.2°): 6.2°, 11.1°, 12.5°, 14.3°, 16.7°, 19.7°, 21.5°, 22.4°, 24.9°, 28.7°.

¹³C-NMR (100 MHz, solid state) δ (± 0.5 ppm): 41.4 ppm, 69.7 ppm, 106.3 ppm, 113.3 ppm, 119.0 ppm, 144.0 ppm, 146.5 ppm, 152.0 ppm, 157.2 ppm, 162.7 ppm.

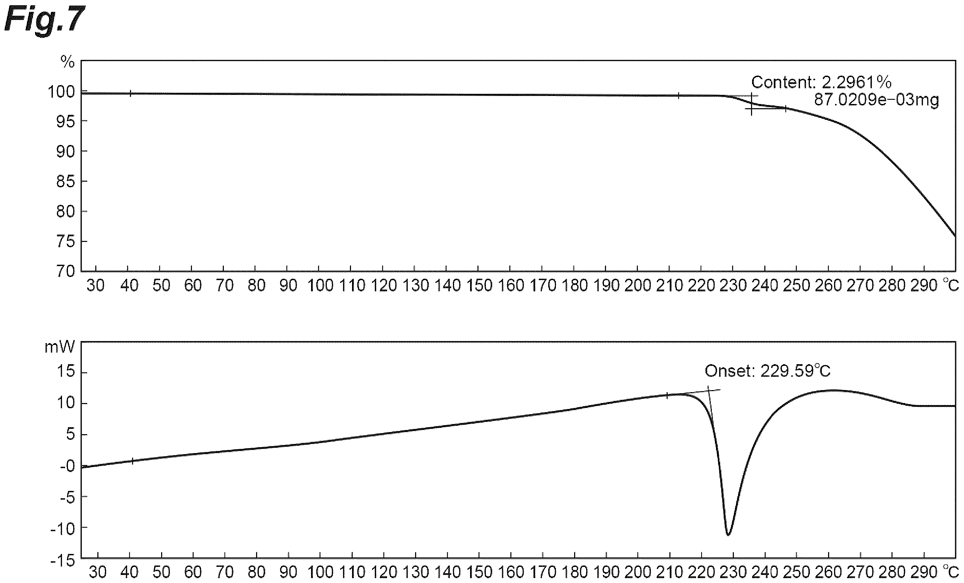

Figure 1, Figure 4, and Figure 7 respectively show the powder X-ray diffraction pattern, ¹³C solid state NMR spectrum, and chart of the thermal analyses TG-DTA for the α-type crystal of a monophosphate of compound (I) obtained with the above method.

### Example 2

### Preparation of β-Type Crystal of Monophosphate of Compound (I)

Compound (I) (approximately 300 mg) was dissolved in acetone (3.5 mL),42 µL of phosphoric acid (1 equivalent to compound (I)) was added dropwise, and the resultant was then stirred at room temperature overnight. Thereafter, 1 mL of heptane was added, the resultant was stirred at room temperature overnight, and a solid precipitated was collected by filtration to afford the title crystal.

Powder X-ray diffraction peaks (transmission method, 2θ ± 0.2°):5.6°, 9.0°, 16.0°, 18.5°, 19.5°, 19.9°, 22.8°, 23.9°

¹³C-NMR (100 MHz, solid state) δ (± 0.5 ppm): 11.2 ppm, 73.2 ppm, 103.2 ppm, 115.0 ppm, 117.9 ppm, 127.7 ppm, 129.3 ppm, 165.5 ppm

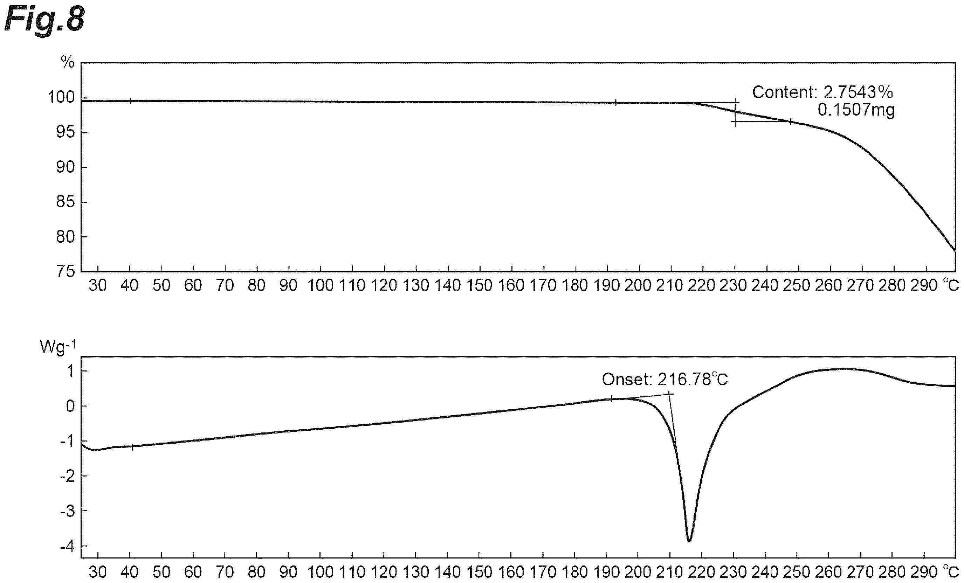

Figure 2, Figure 5, and Figure 8 respectively show the powder X-ray diffraction pattern, ¹³C solid state NMR spectrum, and chart of the thermal analyses TG-DTA for the β-type crystal of a monophosphate of compound (I) obtained with the above method.

### Example 3

### Preparation of Crystal of Monomaleate of Compound (I)

To a solution of compound (I) (6.5 g) in methyl tert-butyl ether (200 mL), a solution of maleic acid (1.573 g) in methyl tert-butyl ether (60 mL) was added dropwise at room temperature over 15 minutes. After the dropwise addition, the resultant was stirred at room temperature overnight, and a salt precipitated was then collected by filtration through filter paper (5 µm), washed with methyl tert-butyl ether (26 mL), and then dried on the filter paper under air flow to afford the title crystal (6.52 g).

Powder X-ray diffraction peaks (transmission method, 2θ ± 0.2°): 8.5°, 9.9°, 17.0°, 18.2°, 19.8°, 20.8°, 22.2°, 22.8°, 24.3°, 25.3°. ¹³C-NMR (100 MHz, solid state) δ (± 0.5 ppm): 13.4 ppm, 15.2 ppm, 40.5 ppm, 73.7 ppm, 98.5 ppm, 105.2 ppm, 132.0 ppm, 140.5 ppm, 164.8 ppm, 170.2 ppm, 172.6 ppm.

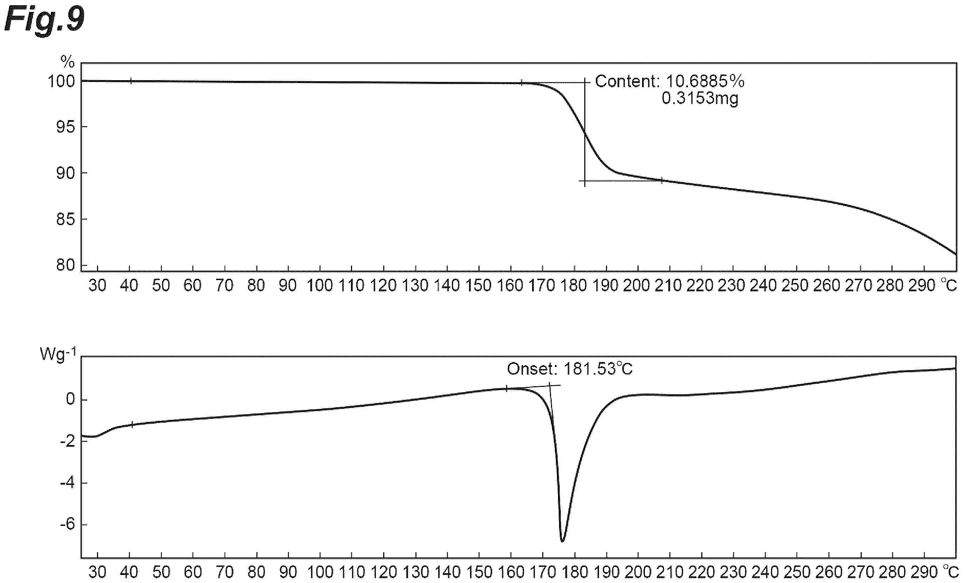

Figure 3, Figure 6, and Figure 9 respectively show the powder X-ray diffraction pattern, ¹³C solid state NMR spectrum, and chart of the thermal analyses TG-DTA for the crystal of the monomaleate of compound (I) obtained with the above method.

### Powder X-Ray Diffraction Measurement

In powder X-ray crystal diffractometry for the crystals obtained in Examples shown above, each crystal obtained was placed on a sample stage of a powder X-ray analyzer, and measurement was performed under the following measurement conditions.

### (Conditions for transmission method)

X-ray source: Cu-Kα
Voltage: 45 kV
Current: 200 mA
Optical system: collector optics
Soller slit: 2.5°
Detector: D/teX Ultra 250 (one-dimensional semiconductor detector)
Scan speed: 10°/min
Step width: 0.01°
Scan range: 3° to 35°
Sample holder: aluminum holder and Mylar film

In ¹³C solid state NMR spectrum for the crystals obtained in Examples shown above, approximately 150 to 300 mg of each solid sample was enclosed in a sample tube, and measurement was performed under the following conditions.

### (Measurement conditions)

Apparatus used: Avance 400 MHz (manufactured by Bruker) 7 mm-CPMAS probe (manufactured by Bruker)
Nucleus measured: ¹³C (resonance frequency: 100.6248425 MHz)
Measurement temperature: room temperature
Pulse mode: CPTOSS measurement
Rotational frequency: 5000 Hz
Pulse-recurrence time: 5 sec
Contact time: 1 msec
Number of scans: 8192
Reference substance: glycine (external reference: 176.03 ppm)

In thermal analysis, each crystal obtained in Examples shown above was precisely weighed on an aluminum sample pan, and measurement was performed under the following conditions.

### (Measurement conditions)

Atmosphere: under 50 mL/min nitrogen gas flow
Control: empty aluminum sample pan
Temperature increase rate: 20°C/min
Sampling interval: 1 sec
Range of measurement temperature: 25 to 300°C

### Pharmacological Test Example

The following pharmacological test was carried out with compound (I).

With use of 3D7 and K1 strains, which are a drug-sensitive strain and drug-resistant strain of Plasmodium falciparum, respectively, provided by The Tropical Disease Research Center, Kitasato University, the in vitro antimalarial activity of the compound for these plasmodia was determined. In culture of the test protozoan parasites, maintenance and subculture were performed with a partially modified version of a method by Trager and Jensen (Trager, W and Jensen, J.: Human malaria parasites in continuous culture, Science, 193:673-677, (1976)), and the resultants were used. Specifically, in a culture flask, erythrocytes with protozoan infection obtained by subculture with RPMI1640 medium to which 10% human plasma and 2% fresh human erythrocytes had been added were diluted (proportion of erythrocytes with protozoan infection: 0.25 to 1%), and subjected to continuous culture by culturing in a mixed gas of 5% O₂-5% CO₂-90% N₂ at 37°C, wherein medium exchange and supplementation with fresh erythrocytes were performed every 2 to 3 days. The protozoan infection rate was maintained within the range of 0.25 to 10%. Drug sensitivity test was carried out with a modified version of a method by Desjardins et al. (Desjardins, R.E., Canfield, C. J., Haynes, D. E. and Chulay, J. D.: Quantitative assessment of antimalarial activity in vitro by a semiautomated microdilution technique. Antimicrob. Agents Chemother., 16:710-718 (1979)). Used as test compounds were compound (I) and the existing antimalarial agents artemisinin and chloroquine, as control compounds. Specifically, to each well of a 96-well plate, 199 µL of a precultured protozoan suspension (hematocrit value: 2%, proportion of erythrocytes with protozoan infection: 0.75 to 1%) and 1 µL of a solution (DMSO solution) of a test compound obtained by serial dilution to attain final concentrations of 1 to 0.001 µg/mL were added and mixed well, and the resultant was then cultured in the above-mentioned mixed gas for 72 hours. Used for assay of protozoan growth was a method of colorimetry of protozoan lactate dehydrogenase (p-LDH), being a modified version of a method by Makler et al. (Makler, M. T., Rise, J. M., Williams, J. A., Bancroft, J. E., Piper, R. C., Gibbins, B. L. and Hinrichs, D. J.: Parasite lactate dehydrogenase as an Assay for Plasmodium falciparum drug sensitivity, Am. J. Med. Hyg., 48:739-741 (1993)). Specifically, the 96-well plate after culture for 72 hours was directly frozen at -20°C over a whole day and night, and then thawed at 37°C to cause the hemolysis of erythrocytes with protozoan infection and break the protozoan parasites; thus, a crude enzyme solution was prepared. To each well of another 96-well plate, 100 µL of an enzymatic reaction mixture (110 mM lithium lactate, 0.5 mM acetylpyridine-adenine dinucleotide, 50 mM Tris (pH 7.5), 10 mM EDTA, 50 mM KCl, and 15 g/L PEG6000) and 20 µL of the crude enzyme solution were added, mixed well, and reacted at room temperature for 30 minutes, and 20 µL of a 1:1 mixed solution of 2 mg/mL nitroblue tetrazolium solution and 0.1 mg/mL phenazine ethosulfate solution was added to each well, and reacted under shading at room temperature for 1.5 hours. Blue formazan, a product generated through the reaction, was detected by measuring absorbance at a measurement wavelength of 660 nm by using a microplate reader to determine the presence or absence of protozoan growth by colorimetry. The 50% protozoan-growth-inhibitory concentration (IC₅₀ value) of each compound was determined with use of a concentration-action curve for the compound. Compound (I) exhibited an IC₅₀ value of 0.026 µg/mL (0.053 µM) for the 3D7 strain of Plasmodium falciparum, and 0.043 µg/mL (0.089 µM) for the K1 strain.

The following drug efficacy evaluation with a humanized mouse malaria model was carried out by The Art of Discovery S. L. (Vizcaya, Spain) with use of compound (I). In short, erythrocytes infected with Plasmodium falciparum were intravenously inoculated into mice with transplanted human erythrocytes, and drug treatment was initiated after the lapse of 72 hours. The therapeutic effect in terms of infection rates was evaluated by determining the proportion of infected erythrocytes in peripheral blood.

Used in the present test was a Pf3D7^{0087/N9} strain of Plasmodium falciparum, which was produced according to study described in Reference (1) shown below. Human erythrocytes were transplanted into female NSG (NOD-scid IL-2Rγnull) immunodeficient mice, and the proportion of human erythrocytes in all the erythrocytes in peripheral blood was maintained to be 40% or more throughout the test period. In RP1640 medium to which 25% inactivated human serum and 3.1 mM hypoxanthine had been added, human erythrocytes were suspended to reach 50 to 75%, and intraperitoneal administration of 1 mL of the suspension or intravenous administration of 0.7 mL of the suspension was performed for each individual of the mice. To each of the thus-obtained humanized NSG mice, 0.3 mL of the blood of an infected mouse, which had been separately prepared and diluted so that 1.17 × 10⁸ infected erythrocytes were contained per 1 mL, was intravenously inoculated. Drug treatment was initiated 72 hours after the infection, and a drug solution in a volume of 10 mL per 1 kg of body weight was orally administered once per day for 3 days. Analysis of the numbers of infected erythrocytes in peripheral blood was carried out according to an approach reported in Reference (2) shown below by using a flow cytometry method, and in the results each proportion of the number of infected erythrocytes to the total number of erythrocytes in peripheral blood was represented as an infection rate. Specifically, 2 µL of peripheral blood collected from an infected mouse was stained with TER-119-Phycoeruthrine (mouse erythrocyte marker) and SYTO-16 (dye for nucleic acid staining), and analyzed by flow cytometry. For therapeutic effect, a daily dose with which the infection rate on day 7 after the infection decreased by 90% from that in a nontreatment group was calculated as ED₉₀, and the ED₉₀ of compound (I) was found to be 3.2 mg/kg/day.
(1) Angulo-Barturen et al. A murine model of falciparum-malaria by in vivo selection of competent strains in nonmyelodepleted mice engrafted with human erythrocytes. Plos One. 2008 May 21;3(5):e2252.
(2) Jimenez-Diaz et al. Quantitative measurement of Plasmodium-infected erythrocytes in murine models of malaria by flow cytometry using bidimensional assessment of SYTO-16 fluorescence. Cytometry A. 2009 Mar;75(3):225-35.

Two possible prophylactic effects are contemplated. Expected as the first is an effect to inhibit the maturation of protozoan parasites from sporozoites, as being injected by a mosquito, into schizonts in liver cells. Reference (3) shown below has reported that MSP1, a representative GPI-anchored protein, is essential for the maturation in liver cells, and GWT1 inhibitors are expected to be effective for protozoan parasites not only in the blood phase but also in the liver phase. Available for providing the evidence for the effect on protozoan parasites in the liver phase is, as described in References (4) and (5) shown below, ex vivo liver cell infection experiment with sporozoites of rodent plasmodia grown in a mosquito. The second possibility is arresting the growth of protozoan parasites in the first infection cycle of the blood phase, which is expected to allow protozoan parasites to be exterminated before the onset of symptoms of malaria. It has been reported in Reference (5) shown below that the prophylactic effect in the blood phase can be confirmed by a mouse model with rodent plasmodia, and carrying out the same experiment after administering a GWT1 inhibitor in advance is expected to allow the prophylactic effect targeting the blood phase to be demonstrated.
(3) Kawabata Y, Udono H, Honma K et al. Merozoite Surface Protein 1-Specific Immune Response is Protective against Exoerythrocytic Forms of Plasmodium yoelii. Infection and Immunity 2002;70:6075-82.
(4) Hovlid ML, Winzeler EA. Phenotypic screens in antimalarial drug discovery. Trends Parasitol. 2016;32:697-707.
(5) Kato N, Comer E, Sakata-Kato T et al. Diversity-oriented synthesis yields novel multistage antimalarial inhibitors. Nature 2016;538:344-349.

## Claims

1. A monophosphate or monomaleate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine- 3-carboxamide represented by the following formula (I):

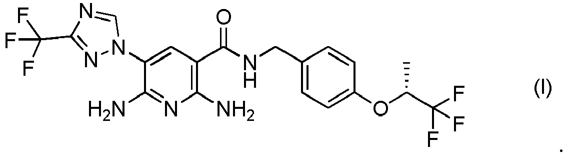

2. A crystal of a monophosphate or monomaleate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine- 3-carboxamide represented by the following formula (I):

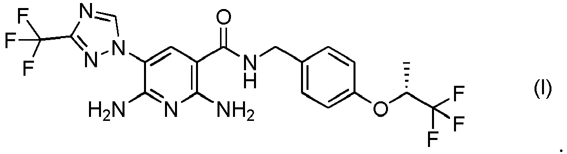

3. An α crystal of a monophosphate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine- 3-carboxamide represented by the following formula (I): 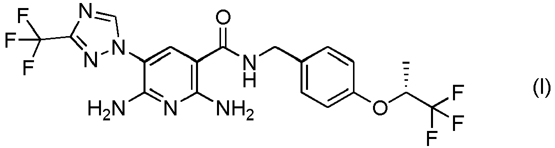 having a diffraction peak at a diffraction angle (2θ ± 0.2°) of 6.2° in a powder X-ray diffraction using CuKα as an X-ray source.

4. An α crystal of a monophosphate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine- 3-carboxamide represented by the following formula (I): 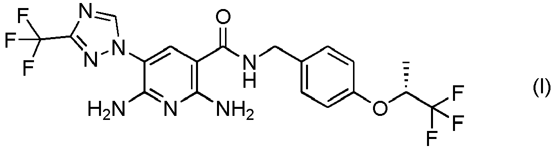 having diffraction peaks at diffraction angles (2θ ± 0.2°) of 6.2°, 12.5° and 14.3° in a powder X-ray diffraction using CuKα as an X-ray source.

5. An α crystal of a monophosphate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine- 3-carboxamide represented by the following formula (I): 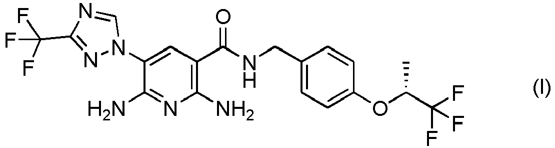 having diffraction peaks at diffraction angles (2θ ± 0.2°) of 6.2°, 11.1°, 12.5°, 14.3° and 24.9° in a powder X-ray diffraction using CuKα as an X-ray source.

6. An α crystal of a monophosphate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine- 3-carboxamide represented by the following formula (I): 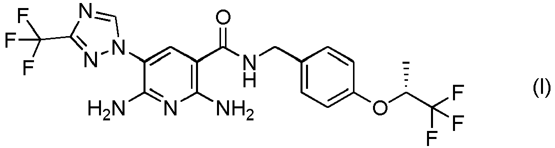 having a powder X-ray diffraction pattern substantially identical to a powder X-ray diffraction pattern shown in Figure 1 in a powder X-ray diffraction using CuKα as an X-ray source.

7. An α crystal of a monophosphate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine- 3-carboxamide represented by the following formula (I): 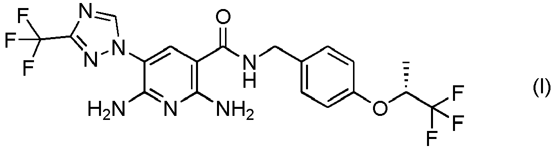 having a peak at a chemical shift (δ ± 0.5 ppm) of 157.2 ppm in a ¹³C solid state NMR spectrum with glycine as an external reference.

8. An α crystal of a monophosphate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine-3-carboxamide represented by the following formula (I): 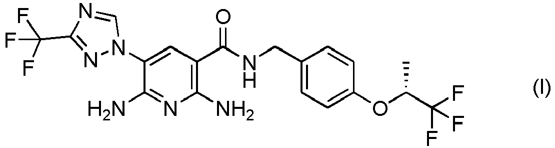 having peaks at chemical shifts (δ ± 0.5 ppm) of 41.4 ppm, 157.2 ppm and 162.7 ppm in a ¹³C solid state NMR spectrum with glycine as an external reference.

9. An α crystal of a monophosphate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine- 3-carboxamide represented by the following formula (I): 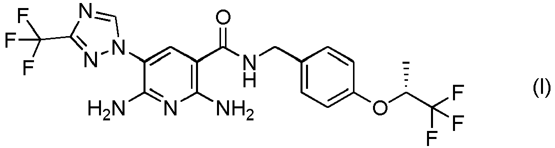 having peaks at chemical shifts (δ ± 0.5 ppm) of 41.4 ppm, 144.0 ppm, 146.5 ppm, 157.2 ppm and 162.7 ppm in a ¹³C solid state NMR spectrum with glycine as an external reference.

10. An α crystal of a monophosphate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine- 3-carboxamide represented by the following formula (I): 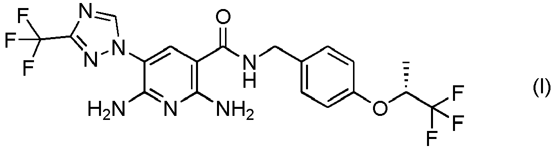 having a ¹³C solid state NMR spectrum substantially identical to a ¹³C solid state NMR spectrum shown in Figure 4 in a ¹³C solid state NMR spectrum with glycine as an external reference.

11. A β crystal of a monophosphate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine-3-carboxamide represented by the following formula (I): 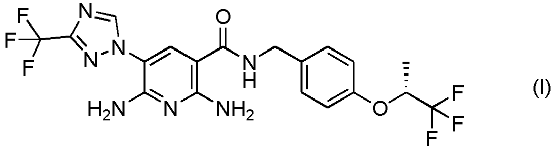 having a diffraction peak at a diffraction angle (2θ ± 0.2°) of 5.6° in a powder X-ray diffraction using CuKα as an X-ray source.

12. A β crystal of a monophosphate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine- 3-carboxamide represented by the following formula (I): 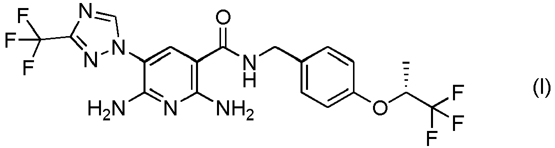 having diffraction peaks at diffraction angles (2θ ± 0.2°) of 5.6°, 9.0° and 16.0° in a powder X-ray diffraction using CuKα as an X-ray source.

13. A β crystal of a monophosphate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine- 3-carboxamide represented by the following formula (I): 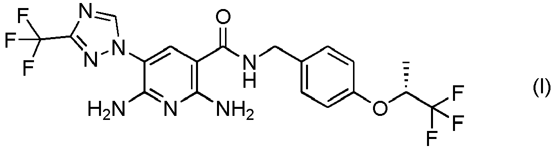 having diffraction peaks at diffraction angles (2θ ± 0.2°) of 5.6°, 9.0°, 16.0°, 22.8° and 23.9° in a powder X-ray diffraction using CuKα as an X-ray source.

14. A β crystal of a monophosphate of 2,6-diamino-5-[3-(trifluoromethyl)-1H-1,2,4-triazol-1 -yl]-N-(4- {[(2R)-1,1,1 -trifluoropropan-2-yl]oxy}benzyl)pyridine- 3-carboxamide represented by the following formula (I): 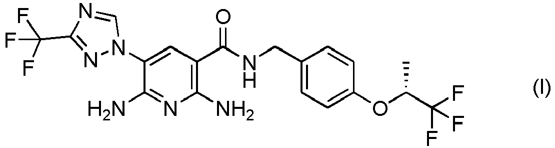 having a powder X-ray diffraction pattern substantially identical to a powder X-ray diffraction pattern shown in Figure 2 in a powder X-ray diffraction using CuKα as an X-ray source.

15. A β crystal of a monophosphate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine- 3-carboxamide represented by the following formula (I): 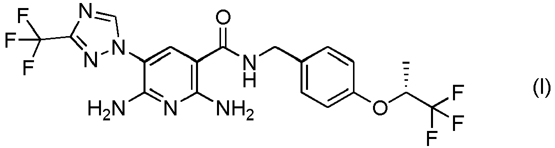 having a peak at a chemical shift (δ ± 0.5 ppm) of 129.3 ppm in a ¹³C solid state NMR spectrum with glycine as an external reference.

16. A β crystal of a monophosphate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine- 3-carboxamide represented by the following formula (I): 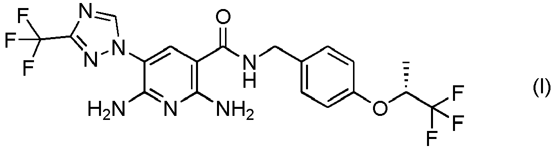 having peaks at chemical shifts (δ ± 0.5 ppm) of 11.2 ppm, 115.0 ppm and 129.3 ppm in a ¹³C solid state NMR spectrum with glycine as an external reference.

17. A β crystal of a monophosphate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine- 3-carboxamide represented by the following formula (I): 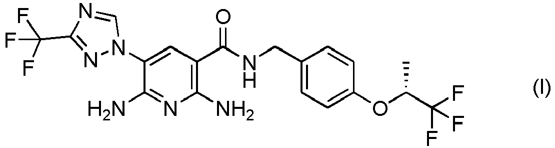 having peaks at chemical shifts (δ ± 0.5 ppm) of 11.2 ppm, 115.0 ppm, 117.9 ppm, 129.3 ppm and 165.5 ppm in a ¹³C solid state NMR spectrum with glycine as an external reference.

18. A β crystal of a monophosphate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine- 3-carboxamide represented by the following formula (I): 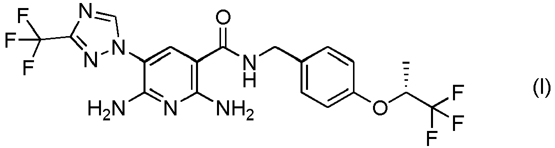 having a ¹³C solid state NMR spectrum substantially identical to a ¹³C solid state NMR spectrum shown in Figure 5 in a ¹³C solid state NMR spectrum with glycine as an external reference.

19. A crystal of a monomaleate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine- 3-carboxamide represented by the following formula (I): 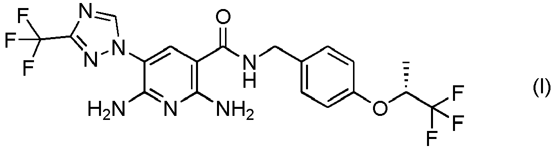 having a diffraction peak at a diffraction angle (2θ ± 0.2°) of 17.0° in a powder X-ray diffraction using CuKα as an X-ray source.

20. A crystal of a monomaleate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine- 3-carboxamide represented by the following formula (I): 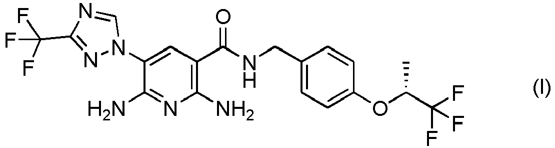 having diffraction peaks at diffraction angles (2θ ± 0.2°) of 17.0°, 18.2° and 25.3° in a powder X-ray diffraction using CuKα as an X-ray source.

21. A crystal of a monomaleate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine- 3-carboxamide represented by the following formula (I): 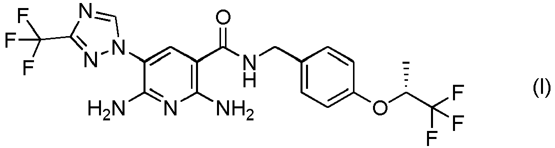 having diffraction peaks at diffraction angles (2θ ± 0.2°) of 8.5°, 9.9°, 17.0°, 18.2° and 25.3° in a powder X-ray diffraction using CuKα as an X-ray source.

22. A crystal of a monomaleate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine- 3-carboxamide represented by the following formula (I): 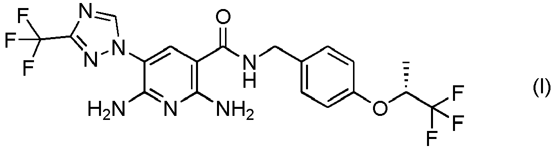 having a powder X-ray diffraction pattern substantially identical to a powder X-ray diffraction pattern shown in Figure 3 in a powder X-ray diffraction using CuKα as an X-ray source.

23. A crystal of a monomaleate of 2,6-diamino-5-[3-(trifluoromethyl)-1H-1,2,4-triazol-1-yl]-N-(4-{[(2R)-1,1,1-trifhioropropan-2-yl]oxy}benzyl)pyridine- 3-carboxamide represented by the following formula (I): 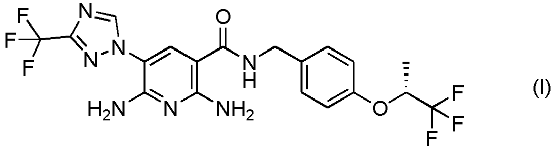 having a peak at a chemical shift (δ ± 0.5 ppm) of 172.6 ppm in a ¹³C solid state NMR spectrum with glycine as an external reference.

24. A crystal of a monomaleate of 2,6-diamino-5-[3-(trifluoromethyl)-1H-1,2,4-triazol-1-yl]-N-(4-{[(2R)-1,1,1-trifhioropropan-2-yl]oxy}benzyl)pyridine- 3-carboxamide represented by the following formula (I): 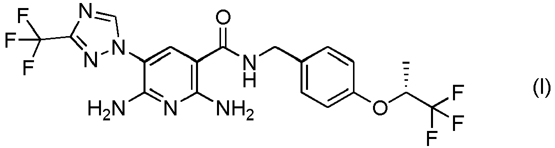 having peaks at chemical shifts (δ ± 0.5 ppm) of 132.0 ppm, 170.2 ppm and 172.6 ppm in a ¹³C solid state NMR spectrum with glycine as an external reference.

25. A crystal of a monomaleate of 2,6-diamino-5-[3-(trifluoromethyl)-1H-1,2,4-triazol-1-yl]-N-(4-{[(2R)-1,1,1-trifluoropropan-2-yl]oxy}benzyl)pyridine- 3-carboxamide represented by the following formula (I): 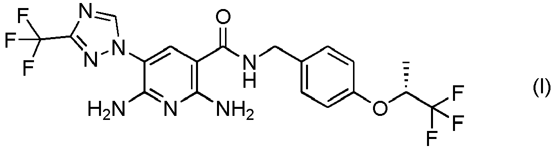 having peaks at chemical shifts (δ ± 0.5 ppm) of 98.5 ppm, 105.2 ppm, 132.0 ppm, 170.2 ppm and 172.6 ppm in a ¹³C solid state NMR spectrum with glycine as an external reference.

26. A crystal of a monomaleate of 2,6-diamino-5-[3-(trifluoromethyl)-1H-1,2,4-triazol-1-yl]-N-(4-{[(2R)-1,1,1-trifhioropropan-2-yl]oxy}benzyl)pyridine- 3-carboxamide represented by the following formula (I): 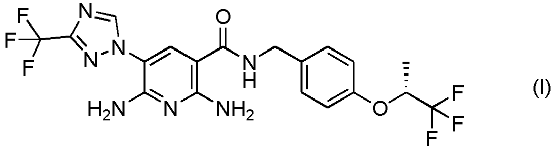 having a ¹³C solid state NMR spectrum substantially identical to a ¹³C solid state NMR spectrum shown in Figure 6 in a ¹³C solid state NMR spectrum with glycine as an external reference.

27. A pharmaceutical composition comprising the salt according to claim 1 or the crystal according to any one of claims 2 to 26.
